# EUROPEAN PATENT SPECIFICATION

(11) **EP 0 439 542 B1**
(45) Date of publication and mention of the grant of the patent: **24.02.1999**
(21) Application number: 89912578.5
(22) Date of filing: 20.10.1989
(51) Int. Cl.: C07K 14/235

(54) **FILAMENTOUS HEMAGGLUTININ OF B. PERTUSSIS**
FASER-HEMAGGLUTININ VON B. PERTUSSIS
HEMAGGLUTININE FILAMENTEUSE DE B. PERTUSSIS

(30) Priority: 27.10.1988 US 263648
(43) Date of publication of application: 07.08.1991
(73) Proprietor: THE BOARD OF TRUSTEES OF THE LELAND STANFORD JUNIOR UNIVERSITY, Stanford California 94305 (US); SCLAVO S.p.A., I-53100 Siena (IT)
(72) Inventor: RELMAN, David, A., Menlo Park, CA 94025 (US); DOMENIGHINI, Mario, I-53100 Siena (IT); RAPPUOLI, Rino, I-53035 Monteriggoni (IT); FALKOW, Stanley, Portola Valley, CA 94025 (US)
(74) Representative: Harrison, David Christopher
(86) International application number: US8904732
(87) International publication number: WO9004641

(56) References cited:
- EP-A- 175 841
- EP-A- 231 083
- EP-A- 235 474
- EP-A- 287 732
- Infection and Immunity, Volume 55, issued January 1987, (BROWN et al) "Cloning of the filamentous hemagglutinin of Bordetella pertussis and its expression in Escherichia coli" see page 154-161, see especially, the Abstract and figure 4.
- Journal of Bacteriology, Volume 170, issued July 1988, (STIBITZ et al) "Genetic analysis of a region of the Bordetella pertussis chromosome encoding filamentous hemagglutinin and the pleiotropic regulatory locus vir", see pages 2904-2913, see especially the Abstract and figure 1.
- FEMS Microbiology Letters, Volume 37, issued September 1986, (MATTEI et al) "Molecular cloning of a coding sequence of Bordetella pertussis filamentous hemagglutinin gene", see pages 73-77, see especially, the Abstract and page 77.
- Proceedings of the Fourth International Symposium on Pertussis, Develop. biol. Standard. Volume 61 issued 1985 (REISER et al) "Bordetella pertussis filamentous hemagglutinin gene: Molecular cloning of a potential coding sequence" see pages 265-271, see especially, the Abstract.
- Proceedings of the National Academy of Sciences, U.S.A., Volume 86, issued April 1989 (RELMAN et al) "Filamentous hemagglutinin of Bordetella pertussis: Nucleotide sequence and crucial role in adherence", see pages 2637-2641, see especially, the Abstract and figure 2.
- MOLECULAR MICROBIOLOGY, vol.4, no., 1990, page 787 - 800, DOMENIGHINI ET AL 'GENETIC CHARACTERIZATION OF BORDETELLA PERTUSSIS FILAMENTOUS HAEMAGGLUTININ'
- INFECTION AND IMMUNITY, vol.65, no.8, 1997, page 3465 - 3468, LIU ET AL 'CHARACTERIZATION OF A RECOMBINANT FRAGMENT THAT CONTAINS A CARBOHYDRATE RECOGNITION DOMAIN OF THE FILAMENTOUS HEMAGGLUTININ'

## Description

This invention relates to the gene encoding filamentous hemagglutinin of B. pertussis, the protein product and the use of the gene and the product for developing vaccines by genetic engineering techniques.

### BACKGROUND

Bordetella pertussis is a small gram negative bacillus found only in humans. It is the etiologic agent of the childhood disease whooping cough, also known as pertussis. In susceptible individuals, the disease may progress to a serious paroxysmal phase. Violent and spasmodic coughing occurs, with the patient being subject to secondary injury from the hypoxia and convulsions attendant with the coughing paroxysms. Secondary infections, encephalopathy and death may occur. The discrete molecular moiety that has been associated with the severe effects in the paroxysmal stage of the disease is pertussis toxin (PTX). PTX has been reported under a variety of names, including lymphocytosis promoting factor, histamine sensitizing factor and islet-activating protein.

Another protein, filamentous hemagglutinin (FHA) is a surface associated protein expressed by B. pertussis under the control of a trans-acting vir locus. FHA, while poorly characterized, is thought to act as a major adhesion and immunodominant antigen in the course of human infection. This protein appears as a heterogeneous collection of polypeptide species on sodium dodecylsulfate-polyacrylamide gel electrophoreses, ranging from approximately 60 to 220 kDa (kilodaltons). It is likely that most of the smaller, commonly seen protein gel bands represent degradation products of a dominant 220kDa species. Electron microscopy of this protein reveals a filamentous structure with dimensions of 2nm by 40-100nm.

It has been suggested that FHA is one of the most important factors mediating the bacterial-eukaryotic cell adhesive interactions. Furthermore, FHA stimulates an immune response in humans following clinical disease and acts as an immunoprotective antigen in a model system employing aerosol challenge of immunized mice. Although less effective than PTX when used alone, FHA and PTX together demonstrate a synergistic immunoprotective effect.

### RELEVANT LITERATURE

A description of the B. pertussis hemagglutinin protein may be found in Irons et al., J. Gen. Microbiol. (1983) 129:2769-2778; Arai and Sato, Biochem. Biophys. Acta (1976) 444:765-782; and Zhang et al., Infect. Immun. (1985) 48:422-427. Physiological properties are described by Tuomanen and Weiss, J. Infect. Dis. (1985) 152: 118-125; Lenin et al., FEMS Microbiol. Lett. (1986) 37:89-94; Urisu et al., Infect. Immun. (1986) 52:695-701; Redd et al., J. Clin. Microbiol. (1988) 26:1373-1377; Oda et al., J. Infect. Dis. (1984) 150:823-833; Robinson and Irons, Infect. Immun. (1983) 40:523-528; Sato and Sato, ibid. (1984) 46:415-421; and Ad Hoc Group for the Study of Pertussis Vaccins, Lancet i (1988) 955-960.

Cloning of the filamentous hemagglutinin structural gene or fragment thereof has been reported by Brown and Parker, Infect. Immun. (1987) 55:154-161; Reiser et al., Dev. Biol. Stand. (1985) 61:265-271; Mattei et al., FEMS Microbiol. Lett. (1986) 36:73-77; Stibitz et al., J. Bacteriol. (1988) 170:2904-2913 and in European Patent Application No. 0235474 (Institut Pasteur).

Brown and Parker disclose the cloning of a DNA fragment from B. pertussis, said to contain the whole fhaB gene which was mapped to a 6.5kb region of an 8.6kb fragment. Expression of the fragment in E.coli yielded proteins recognised by fhaB - specific antibodies.

Mattei et al disclose the cloning of a 2.9kb DNA fragment from B. pertussis which was expressed to give a fusion protein recognised by anti-FHA antibodies. The fragment was partially sequenced.

Stibitz et al disclose the cloning of a large DNA fragment containing the vir and fhaB genes. The fhaB gene was mapped to a 6kb region just downstream of the vir gene.

EP-A-0235474 discloses the cloning of a DNA fragment from B. pertussis and the expression of a fusion protein recognised by anti-FHA antibodies. Also discussed is the possible use of such expression products in vaccines and the effect of anti-FHA antibodies in the prevention of B. pertussis binding to fibroblast cells.

Chemical analysis of the filamentous hemagglutinin has been reported by Sato et al., Infect. Immun. (1983) 41:313-320.

### SUMMARY OF THE INVENTION

DNA sequences encoding at least a portion of the B. pertussis fhaB gene, genetically engineered products including such sequences, the expression products of such sequences, and cells containing such genetically engineered sequences are provided for use in the diagnosis, prophylaxis and therapy of whooping cough.

### DESCRIPTION OF THE SPECIFIC EMBODIMENTS

The subject invention concerns nucleotide sequences associated with the filamentous hemagglutinin protein of B. pertussis and their use in the diagnosis prophylaxis and therapy of whooping cough or pertussis.

According to the invention there is provided a nucleic acid sequence of less than 15kb comprising the 10,788 kb *B.pertussis fha*B gene free from the *fha*A gene and a nucleic acid sequence of less than 15kb comprising a sequence of nucleotides encoding the *B.pertussis* FHA protein of 3597 amino acids. The nucleic acid sequence is in one aspect nucleotides 253 to 11,040 as set out below.

The invention further provides a fragment of a nucleic acid sequence of any preceding claim encoding an approximately 230kDa portion of the FHA protein, said portion being the N-proximal portion of said protein.

The invention also provides a fragment of a nucleic acid sequence of the invention which encodes a peptide of at least 12 amino acids in length, preferably a fragment of 100 bp, said fragment being a fragment of the open reading frame of the *FhaB* gene which encodes the peptide encoded by the sequence set out below, wherein said nucleotides are characterised by a motif selected from the group:
a) nucleotides encoding the RGD cell recognition sites at positions 1097 or 2599 of the FHA polypeptide;
b) nucleotides encoding the RRARR proteolytic site at position 1069 of the FHA polypeptide;
c) nucleotides encoding direct repeats located between nucleotides 1468 and 1746 of the *FhaB* gene;
d) nucleotides encoding the motif EARKDE of positions 1211 to 1216 of the FHA polypeptide;
e) nucleotides encoding the motif SKQDER at positions 3075 to 3080 of the FHA polypeptide;
f) nucleotides encoding the (PK)₅ repeat which repeat begins at residue 3477 of the FHA polypeptide; and
g) nucleotides encoding the sequence VEVVPRKVET starting at positions 3319 and 3350 of the FHA polypeptide.

The invention also provides isolated polypeptide fragments of FHA encoded by the open reading frame of these nucleic acid fragments.

In a particularly preferred aspect, the invention provides an isolated polypeptide of at least 12 amino acids in length which is a fragment of the *B.pertussis* FHA protein of 3597 amino acids and which consists essentially of the amino acid sequence E-A-R-K-D-E together with its adjoining sequences up to 100 amino acids in either direction.

Other features of the invention include expression constructs, transformed prokaryotic cells and the use of such cells for producing a polypeptide encoded by a nucleic acid of the invention. Also provides is a vaccine comprising a polypeptide recognized by antibodies to the filamentous hemagglutinin of *B.pertussis,* and the use of polypeptides of the invention in a method of treatment or diagnosis of the human or animal body.

The open reading frame of the B. pertussis fhaB gene is about 10 kbp (specifically about 10788 bp) as the sequence set forth in the experimental section. It encodes a protein of about 368 kDa (about 3597 amino acids), comprising an N-proximal fragment of 230 kDa, which N-proximal fragment is divided by proteolysis into two polypeptide fragments of about 98 and 140 kDa at an arginine-rich peptide sequence RRARR, which are the N-terminal and C-terminal fragments, respectively. This sequence may act as a proteolytic cleavage site. The overall polypeptide is basic, has a relatively high charge density, a pK_{I} of 9.65 and a net charge of +19. Alanine and glycine constitute 27% of the total residues, while only 3 upstreams are present. The last 350 amino acids provide a highly basic region (charge +32; pK_{I} 11.3) rich in proline (17%). At amino acid position 1097 (defined by the start of translation at 253bp from the left-hand EcoRI site) and again at position 2599 is the tripeptide sequence RGD. This sequence is known as a "cell recognition site" for the interaction of fibronectin and other eukaryotic extra-cellular matrix proteins with certain eukaryotic cell receptors, particularly mammals, and may function in a similar manner in FHA mediated bacterial adherence.

The gene appears to be located adjacent to the vir locus. In the direction defined by transcription an apparent regulatory gene fhaA lies about 2-5 kb downstream from fhaB, followed by the gene fhaC, also believed to be a regulatory gene, again in the downstream direction from fhaA. The beginning of the ORF is separated by approximately 430 bp from the first of the bvg genes bvgA. The gene begins at position 253 from the left at the pDR₁ EcoRI site and ends at position 11041 with a TAG codon.

The fhaB gene is characterized by having a high GC content, namely about 67.5%. In addition, there is a series of tandem direct nucleotide repeats of the pattern ABABA in the region from nucleotide 1468 to nucleotide 1746, with the G of the sequence reported in the Experimental section being nucleotide 1. An unusual alternating repeat (PK)₅ begins at residue 3477. The sequence VEVVPRKVET at position 3319 is repeated at position 3350. Transcriptional initiation appears to occur 70-75 bp upstream of the ORF.

Fragments of the open reading frame of at least about 15 bp, more usually at least about 50 bp, and usually at least about 100 bp may find use in a variety of ways. The fragments may be used for diagnostic purposes, as probes in hybridizing to DNA or RNA for detecting the presence of B. pertussis or the like. Use of Southerns, Northerns, dot-blot, or other techniques may be employed. The fragments may be used for encoding peptides of at least about 9 amino acids (27 bp) usually at least about 12 amino acids.

The fragments may also be used in the antisense direction to modulate the amount of the expression product of the fhaB gene, where such modulation may be of interest. Thus, the infectious ability of the organism may be modulated and/or attenuated by reducing the presence of the filamentous hemagglutinin protein on the surface of the organism.

Fragments of interest of the fhaB gene include those fragments associated with the expression of the 98 kDa protein and the 140 kDa protein. Using the numbering as set forth in the sequence provided in this application, the fragment for the 98 kDa protein would terminate between nucleotides 3402 and 3502, usually between 3451 and 3474. The 140 kDa protein is initiated in that region and terminates at about nucleotide 9624. When FHA is originally isolated and purified from B. pertussis liquid culture supernatant using standard techniques there are often 3-4 bands seen on SDS-PAGE, with polypeptide species of 230, 140, 125 and 98 kDa. With increasing time of storage, two new species appear, 75 and 58 kDa with concurrent fading of the 230 kDa band and intensification of the 125 and 98 kDa bands. An identical N-terminal sequence is observed for the 140 and 125 kDa fragments: A-L-R-Q-D-F-F-T-P-G-S-V-V-V-R-A-Q-G-N. This peptide is encoded begining at position 1074, immediately downstream from a proposed proteolytic cleavage site R-R-A-R-R, and terminating at position 1131. Also of interest is the repeat sequence, where the sequence should have at least two repeats, preferably three repeats, and the fragment will be at least about 60 nucleotides, more usually about 100 nucleotides, and may be 278 nucleotides or more, usually not exceeding about 300 nucleotides of the open reading frame, the latter encompassing the entire repeat region. The repeats do not have perfect homology, but show a high degree of conservation.

Regions of interest will be those encoding amino acid sequences 1211 to 1216 (E-A-R-K-D-E), 1876 to 1881 (R-K-D-E-H-R) and 3075 to 3080 (S-K-Q-D-E-R), and adjoining amino acid sequences, extending up to 100 amino acids, usually up to 50 amino acids in either direction, but particularly including at least 3 amino acids of the sequences described above. DNA sequences of interest may include fragments of 3490 to 3590, 3840 to 3940, 5840 to 5940, 9440 to 9540, and fragments of at least 15bp, more usually at least 25bp thereof. The fragment from about 5625 to 5780 does not appear to have any features of interest and may be excluded, unless joined to one of the fragments indicated above.

Antisera prepared against the B. pertussis FHA protein cross-reacts with polypeptide species of B. parapertussis and B. bronchiseptica. Antisera binding to the expression products of the regions 2836-3786 nt, 5212-7294 nt and 6393-8080 nt bound to peptides of parapertussis, while only the antisera of the first two bound to peptides of brochiseptica.

The subject protein or any portion thereof may be prepared in any convenient host, preferably prokaryotic. By transforming an appropriate host with the expression construct, the host will express the polypeptide of interest, which may then be isolated or, as appropriate, the host may be isolated containing the subject protein or portion thereof and used as a vaccine.

The expression construct or cassette will employ a transcription initiation region, the structural gene for the polypeptide to be expressed, and and a transcriptional termination region. The transcriptional initiation region may include only the RNA polymerase binding site or may also include an enhancer or operator to provide for increased expression of the subject protein or portion thereof, or inducible expression of the subject protein or portion thereof.

A large number of transcription initiation regions are known which are active in one or more prokaryotic hosts, such as the lambda left or right promoters, the lac promoter, the trp promoter, the tac promoter, omp promoter, metallothionein promoter, etc. The natural promoter may also find use. The particular promoter will be chosen to provide for efficient expression in accordance with the selection of the host cell line.

For the most part, prokaryotic host cell lines will be used to provide for efficient expression of the filamentous hemagglutinin or portion thereof, integrity of the expression product, ease of isolation of the expression product, and in some situations, the ability to use the host without isolation of the protein, using the transformed host as the vaccine. Various organisms may be used which may provide for an immune response not only to the subject proteins or portions thereof, but also to other pathogens, so that the vaccine will result in immune protection, not only against the B. pertussis organism but also against disease caused by other pathogens.

Various host organisms which may be used include various gram negative organisms, such as E. coli, Salmonella, Yersinia, Pseudomonas, Bordetella, such as the species avium, bronchiseptica, para- pertussis and pertussis, where the last two are particularly preferred.

A previously indicated sequence analysis of the subject protein indicates a guanine plus cytosine content considerably higher than that of the traditional E.coli cloning host (approximately 50%). Therefore, for the most part, the host will desirably have a high guanine plus cytosine content in its genome, preferably at least 60%, more preferably 65%. However, one may use synthetic portions to reduce the ratio of guanine and cytosine for use in organisms lacking a preference for GC.

Various replication systems are available for use in the various host species. For the most part, the vectors will include not only a functional replication system but a marker for selecting transformants comprising the subject structural gene or portion thereof. While it is usually desirable to employ either a plasmid or virus which is stably maintained as a vector without lysogeny, to enhance the efficiency of expression by having a multicopy replication system which is stable in the host, this is not necessary. Thus, one can transform with bare DNA comprising the expression cassette in combination with a marker for selection, where the marker may be joined to the expression cassette or be independently present in the transformation media. In some situations, a vector will be employed which does not have a stable replication system for the expression host. In this manner, selection can be carried out to insure that integration has occurred by selecting for those cells containing the marker.

A wide variety of markers may be used which include antibiotic resistance, resistance to heavy metals, imparting prototrophy to an auxotrophic host, or the like. The particular choice of marker is not critical to this invention, but will be selected for efficiency in selection and efficiency in production of the subject protein or portion thereof.

Depending on the manner of transformation, as well as the host, various other functional capabilities may be provided in the vector. For example, transfer capability may be provided which allows for conjugation in conjunction with a helper plasmid, where once transferred to the recipient host, the vector may no longer be transferred to other hosts. For example, the rlx sequence may be employed, particularly from the P-1 incompatibility group. In addition, the cos site may be employed from bacteriophage lambda. Other markers of interest may include a gene which renders an antibiotic resistant strain sensitive.

The termination region is not critical to this invention and any convenient termination region may be used. The native termination region may be employed or a termination region which is normally associated with the transcription initiation region or a different region. The fact is that many transcription termination regions have been employed and are generally available and may be used with advantage.

The host may be transformed in any convenient way. By using bare DNA, calcium phosphate precipitated DNA may be employed for transformation. Alternatively, conjugation may be employed using a helper plasmid, where a transfer gene is provided in a vector. In some instances, it may be desirable to employ a bacteriophage vector, where the host cell will be transduced or transfected. The technique for introducing the expression cassette comprising the subject gene or portion thereof is not critical to this invention and various alternative protocols find ample exemplification in the literature.

The subject gene may also be subject to various lesions or mutations. For example, the sequence RRARR may be substituted, deleted, or modified so as to remove the peptidase cleavage site. Thus, the protein would be retained substantially intact, with the two potential fragments fused together. This protein could find a variety of uses. Other mutations may include the removal of the upstream portion of the gene, so as to leave only the sequence that is downstream from the RRARR sequence, where an initiation codon may be introduced at the appropriate site. In addition, mutagenesis of an RGD region may cause altered interactions with eukaryotic target cells and perhaps an altered host immune response, both of which may prove useful for disease therapy or prophylaxis.

Mutation can be achieved in a variety of ways using in vitro mutagenesis, primer repair, the polymerase chain reaction, restriction site deletions, insertions, or the like. The particular manner in which the subject gene is modified is not critical to this invention and any conventional technique may be employed which provides for the desired substitutions, deletions or insertions.

The subject gene can be obtained by EcoRI digestion of the plasmid pUW21-26. The resulting 10 kb EcoRI fragment contains the open reading frame of 9375 bp. This fragment may be manipulated at its 5' terminus in a variety of ways. By employing Bal 31 digestion, the sequence may be resected to remove all or a portion of the non-coding region 5' of the initiation codon. Alternatively, one may restrict either upstream or downstream from the initiation codon, where the nucleotides removed by restriction downstream from the initiation codon may be replaced with an appropriate adapter. In this manner, the subject sequence may be inserted into a polylinker downstream from a transcriptional initiation regulatory region and be under the transcriptional initiation regulation of such region.

The subject compositions, both nucleotides and proteins, may find both diagnostic and therapeutic use. For diagnostic use, as already indicated, the sequences may be used to detect the presence of nucleic acid sequences which duplex with the subject sequences as indicative of the presence of B. pertussis. Alternatively, the protein or portion thereof may be used in diagnostic assays, as a labeled or unlabeled reagent for detection of antibodies to the filamentous hemagglutinin in a blood sample or the presence of filamentous hemagglutinin protein in a blood or tissue sample.

Intact protein or portion thereof may be used to prepare antibodies which may be used in diagnosis, prophylaxis or therapy. The antibodies may be polyclonal or monoclonal, preferably monoclonal. Desirably, neutralizing antibodies will be obtained. Antibodies may be mouse antibodies, human antibodies, chimeric antibodies, e.g., mouse variable region and the human constant region, or the like. Of particular interest are those constant regions which bind to complement, such as IgM and IgG isotypes. The antibodies may be used for passive immunization or for treatment in accordance with conventional ways.

The subject compositions also find use as vaccines, as the protein, by itself or in combination with other proteins, e.g., acellular compositions, as cellular compositions in a pertussis or non-pertussis host, in purified or semi-purified form or the like. Desirably, the subject compositions are used in conjunction with a modified pertussis toxin, where the toxin no longer has ADP-ribosyltransferase activity, particularly subunit A. This can be achieved by using ptx3201 as described in Black et al. , Science (1980) 240:656-659. By introducing the subject gene under the transcriptional initiation regulatory control of a constitutive promoter or an inducible promoter, which is not regulated by the normal pertussis transcriptional regulation of the filamentous hemagglutinin gene, one can provide for the enhanced presence of the subject protein on the surface of the B. pertussis cell. In this way, an enhanced immune response may be achieved in response to vaccinating either live or dead organisms.

Because of the various ways in which the subject composition may be administered, the amount administered will vary widely. In addition, the amount of the vaccine will vary in accordance with the nature of the administration, the frequency of the administration, the presence or absence of antigen, the nature of antigen, or the like

The manner of administration may be oral, peritoneal, subcutaneous, intravascular or the like. Usually, an inert carrier is employed, such as sugar, water, aqueous ethanol, phosphate buffered saline, saline, or the like. Adjuvants include aluminum hydroxide, vegetable oils, bacterial toxins, etc. The amount of the active ingredient will generally be in the range of about 25 to 75 µg/kg for a single human dose. Pertussis vaccines have been used previously, and prior usage may be used as a guide for the dosage employed. See, for example, Developments in Biological Standardization, supra.

The following examples are offered by way of illustration and not by way of limitation.

### EXPERIMENTAL

### Materials and Methods

Bacterial Strains and Plasmids. B. pertussis strain BP536 is a spontaneously-occurring streptomycin resistant mutant of the virulent phase (I) parental strain BP338. BP537 is an avirulent phase variant of BP536. The isolation of the Tn5 mutant BP353 has been previously described, Weiss et al., Infect. Immun. (1983) 42:33-41; the transposon insertion site has been mapped more recently (Stibitz et al., 1988, supra) BP338 Tn5-25 carries a Tn5 insertion mutation within the 2.4 kb BamHI segment of fhaB (Stibitz et al., 1988, supra). BP-TOX6 (available from R. Rappuoli) is a derivative of BP536 with a deletion of the pertussis toxin operon and the substitution of a kanamycin resistance cassette at that location. BP-B52 (available from F. Mooi) is a BP536 derivative which carries insertion mutations which inactivate the fim2 and fim3 genes independently. E. coli strains JH101 and SM10 have been described elsewhere (Messing, Recomb. DNA Tech. Bull. (1979) 2:43-48; Simon et al., Bio/Technology (1983) 1:784-791). Cosmid pUW21-26 is a derivative of pHC79 (Hahn and Collins, Gene (1980) 11:291-298) with an approximately 45 kb insert, containing the cloned vir and fha loci from BP338 (Stibitz, 1988, supra). The construction of plasmid vector pRTP1 has been described (Stibitz et al., Gene (1986) 50:133-140).

### Cloning of fhaB and Construction of fhaB Deletion Mutants.

The filamentous hemagglutinin (FHA) structural gene, fhaB, was cloned on a 10 kb EcoRI fragment from cosmid pUW21-26 into the vector pRTP1, creating the recombinant plasmid pDRl. An in-frame partial deletion of fhaB was constructed by re-ligating a pool of BamHI partial digests of pDRl. Plasmids were screened for the loss of an internal 2.4 kb BamHI fragment. The resultant plasmid was designated pDR101.

### Bacterial Conjugations and Allelic Exchange

The technique for conjugal transfer of pRTP1 derivatives from E. coli to B. pertussis has been described (Stibitz et al., 1986, supra). The partially deleted copy of fhaB was exchanged for the wild type allele in B. pertussis BP536 in two steps. First, the E. coli donor, SM10(pDR101), was mated with a B. pertussis recipient, BP536 Tn5-25, which carries a selectable marker within the fhaB fragment to be deleted. Sm^{R} Ap^{R} exconjugants were then plated on media containing Sm alone and screened for the loss of Km resistance, indicating a second crossover event and acquisition of the mutant allele.

### DNA Sequencing and Sequence Analysis

The 10 kb EcoRI fragment containing fhaB was subcloned as three separate BamHI fragments as well as random one to three kb Sau3A fragments in M13mp18 and M13mp19 (Yanisch-Perron et al., Gene (1985) 33:103-119), pEMBL18 and -19 (Dente et al., Nucleic Acids Res. (1983) 11:1645-1655), or Bluescript (Stratagene, San Diego, CA) vectors. DNA inserts were sequenced by the dideoxy chain-termination method (Sanger et al., Proc. Natl. Acad. Sci. USA (1977) 74:5463-5467), using either Klenow fragment or Sequenase (U.S. Biochemical Corporation, Cleveland, Ohio). Synthetic oligonucleotide primers were designed in order to extend sequence reading across large cloned inserts. Assembly of the nucleotide sequence was performed using the software package of the University of Wisconsin Genetics Computer Group (Madison, WI). Further analysis of the completed nucleotide and predicted peptide sequences was performed, using both this package as well as PC/GENE (Intelligenetics, Mountain View, CA).

### Hemagglutination

The ability of B. pertussis strains to agglutinate sheep erythrocytes was assayed in conical pointed-bottom wells of polystyrene Microtiter plates (Dynatech Laboratories, Alexandria, VA). The strains were grown for two to three days on Bordet-Gengou plates, washed twice in phosphate-buffered saline, and resuspended to an OD₆₀₀ of 10 (1.7x10¹⁰ cells/ml). The first well of a microtiter plate received 100µl of this cell suspension, following which the bacteria were two-fold serially diluted 11 times. Sheep erythrocytes were added to each well as 50 µl of a 0.5% PBS-washed suspension. The plates were left at room temperature for three to four hours during which time nonagglutinated erythrocytes slid down the well bottoms forming a dark pellet. Hemagglutinating (HA) activity was expressed as the inverse of the highest dilution without significant pellet formation.

### Western Immunoblots

Polyacrylamide gel electrophoresis was performed in the presence of sodium dodecylsulfate with a 10% separating gel and 20µl of boiled (OD₆₀₀=10) B. pertussis cell suspension with sample buffer. Transfer of protein to nitrocellulose membrane followed the procedure of Towbin et al, Proc. Natl. Acad. Sci. USA (1979) 76:4350-4354. Non-specific antibody binding to the membrane was blocked by pre-incubation with a solution of PBS and 1% nonfat dry milk. Immunological detection of FHA was performed using a 1:1000 dilution of a mixture of (1-54, 1-199, 31E2, 22F10, and 68A6) monoclonal anti-FHA antibodies (obtained from F. Mooi), followed by incubation with a 1:250 dilution of horseradish peroxidase-conjugated goat anti-mouse antisera. HRP activity was detected using a tetramethylbenzidine-containing reaction mixture. fim2 and fim3 production were detected using the same technique and monoclonal antibodies (21E7 and 8E5) specific for these two proteins (obtained from F. Mooi).

### Southern Hybridization

B. pertussis chromosomal DNA was isolated, digested with restriction endonucleases, and separated by agarose gel electrophoresis according to standard techniques (Maniatis et al. (1982), Molecular Cloning: A Laboratory Manual, Cold Spring Harbor Laboratory, Cold Spring Harbor, NY). Transfer of fragments to nitrocellulose followed the method of Smith and Summers (Anal. Biochem. (1980) 109:123-129). Hybridization with probe occurred at 37°C, with 50% formamide and 5xSSC. Membranes were washed twice with 2xSSC at 25°C, twice with 0.1xSSC at 25°C, and then twice with 0.1xSSC at 65°C.

### In vitro Bacterial Adherence

B. pertussis strains were grown on plates for two days and then washed twice in phosphate-buffered saline (PBS). 20µl of bacterial suspension (OD₆₀₀=10) was added to tissue culture plate wells containing 200 µl of MEM and a cover slip on which approximately 5x10⁴ Chinese Hamster Ovary cells had been innoculated and allowed to grow overnight. After incubation at 37°C, 5%CO₂, for four hours, each well was washed vigorously with PBS three times. Any remaining bacteria and CHO cells were fixed with methanol and then stained with Giemsa. All bacterial strains were studied in duplicate and all experiments repeated at least twice. Bacteria adherent to a single CHO cell were counted visually and the mean with standard deviation determined for each strain. Joint 95% confidence intervals were computed based on central limit theorem approximations and Bonferoni techniques.

### Results

### Identification and Cloning of the FM Structural Gene

Previous work had led to the isolation of a cosmid clone, pUW21-26, which hybridized with both vir and fha DNA probes (Stibitz, et al., 1988, supra). The analysis of Tn5 insertion mutations within this cosmid, using FHA colony and Western immunoblots, had suggested that the FHA structural gene, fhaB, was located on a 10 kb EcoRI fragment just to the right of the vir locus. Furthermore, fhaB transcription was believed to begin near the left-hand EcoRI site and proceed from left to right, based upon the correlation of FHA truncated product size with location of the corresponding Tn5 insertion site.

Deletion of the internal 2.4 kb BamHI fragment of fhaB was performed as described above and the mutation returned to the B. pertussis chromosome, yielding strain BP101. The structure of the resultant fhaB mutant locus in this strain was confirmed by Southern blot analysis. The largest FHA cross-reactive polypeptide produced by BP101 measures approximately 150 kDa, as determined by Western blot technique. This truncated FHA product has no hemagglutinating activity.

These data confirmed that the structural gene for FHA must be contained on the 10 kb EcoRI insert of pDRl. This fragment was, therefore, subcloned for dideoxy single-stranded DNA sequencing.

### Construction of fhaB fusion proteins

Seven portions of the fhaB ORF were each cloned into the expression vector pEX34. The result in each case was a translational fusion with the first 98 amino acids of the phage MS2 RNA polymerase. Fusion proteins were expressed in an E. coli host and then purified using preparative SDS-PAGE. One reason for the construction of these fusion proteins was to confirm the absence of a translational stop codon in various regions of the ORF. This aim was addressed by comparison of measured fusion protein molecular weights with those theoretically expected from translational read-through of the entire cloned fhaB inserts. Table 1 lists the fusion proteins with the nucleotide coordinates of the respective fhaB inserts: these data confirm the absence of a stop codon in all of these fhaB fragments.

**Table 1**

| | Observed MW | FRAGMENT | |
|---|---|---|---|
| protein H1 | 45 Kda | BamHI-RsaI | 2836-3786 |
| | | | |
| protein H2 | 85 Kda | BamHI-NruI | 5212-7294 |
| | | | |
| protein H3 | 77 Kda | PvuII-PvuII | 6393-8085 |
| | | | |
| protein H4 | 80 Kda | PvuII-BamHI | 8085-9922 |
| | | | |
| protein H5 | 55 Kda | StuI-BamHI | 8752-9922 |
| | | | |
| protein H6 | 32 Kda | EcoRV-BamHI | 9462-9922 |
| | | | |
| protein H7 | 56 Kda | BamHI-ClaI | 9922-11666 |

### Western immunoblot analysis using fusion protein antisera

Antisera to each of the seven fusion proteins were prepared by intraperitoneal immunisation of mice and were used for two purposes: to correlate each of the FHA SDS-PAGE bands with a region of the fhaB ORF, and to determine what portions of ORF-encoded polypeptide are present in whole Bordetella sp. extracts. Table 2 shows the results of Western immunoblots using each of the seven fusion protein antisera and an FHA protein gel pattern.

The combination of these data with the results of N-terminal amino acid sequencing suggest an origin for the different FHA polypeptide species. The stimulation of a murine polyclonal response by each of the fhaB fusion proteins also argues that FHA contains numerous immunogenic domains.

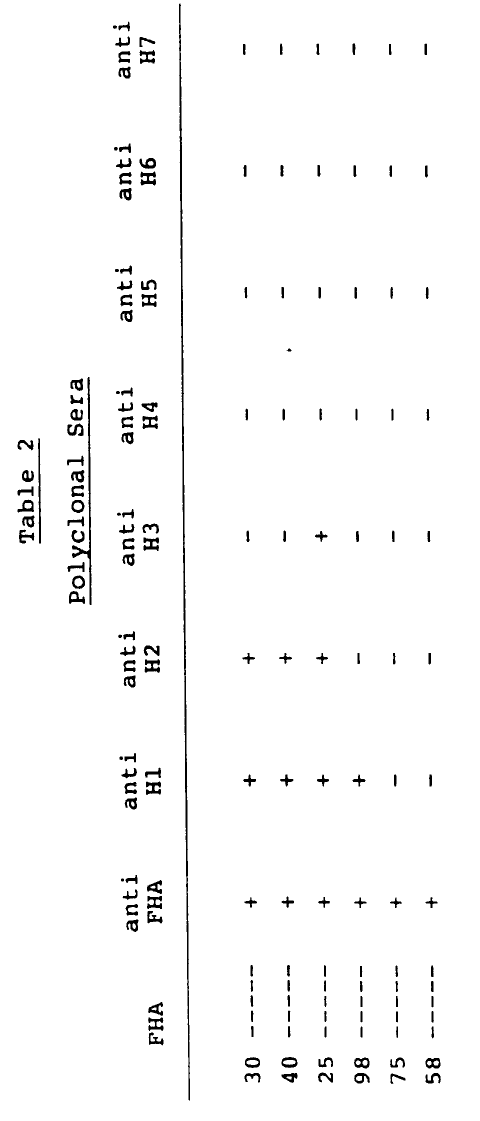

### Nucleotide Sequence of the FM Structural Gene

The sequencing strategy described above yielded a 10036bp-long nucleotide sequence for the EcoRI fragment. Computer analysis identified an open reading frame (ORF) 10788 bp long beginning at an ATG translational start codon 253 bp from the left-hand EcoRI site. Two other in-frame ATG codons are located 45 and 174 bp after the beginning of the ORF; at approximately the position of the third ATG codon begins the use of codons strongly preferred by B. pertussis (defined by B. pertussis pertussis toxin operon codon usage and the UWGCG codon preference program; Gribskov et al., Nucleic Acids Res. (1984) 12:539-549). The ORF and preferred codon usage end at a TAG stop codon 11041 bp from the left-hand EcoRI site. This ORF encompasses the FHA structural gene fhaB; the sequence of the ORF is shown below.

GAATTCCTGCGCTGGCACCCGCGGCGGGCCGGGGAGCGGGTTGTCGGCGCA 51

CGCCTATACGTGCCGGACAGGGTTTGATGGTTTGACTAAGAAATTTCCTAC 102

AAGTCTTGTATAAATATCCATTGATGGACGGATCATTACTGACTGACGAA 153

GTGCTGAGGTTTATCCAGACTATGGCACTGGATTTCAAAACCTAAAACGAG 204

CAGGCCGATAACGGATTCTGCCGATTACTTCACTTCGCTGGTCGGAATATG 255
Met

AACACGAACCTGTACAGGCTGGTCTTCAGCCATGTTCGCGGCATGCTTGTT 306
AsnThrAsnLeuTyrArgLeuValPheSerHisValArgGlyMetLeuVal

CCCGTGAGCGAGCATTGCACCGTCGGAAACACCTTCTGTGGCGCACGCGT 357
ProValSerGluHisCysThrValGlyAsnThrPheCysGlyArgThrArg

GGTCAAGCGCGAAGTGGGGCCCGCGCCACGAGCCTGTCCGTAGCGCCCAAT 408
GlyGlnAlaArgSerGlyAlaArgAlaThrSerLeuSerValAlaProAsn

GCGCTGGCCTGGGCCCTGATGTTGGCGTGTACGGGTCTTCCGTTAGTAACG 459
AlaLeuAlaTrpAlaLeuMetLeuAlaCysThrGlyLeuProLeuValThr

CACGCCCAGGGCTTGGTTCCTCAGGGGCAGACACAGGTGCTGCAGGGCGGG 510
HisAlaGlnGlyLeuValProGlnGlyGlnThrGlnValLeuGlnGlyGly

AACAAGGTTCCCGTTGTCAATATCGCCGACCCAAATTCCGGCGGCGTCTCG 561
AsnLysValProValValAsnIleAlaAspProAsnSerGlyGlyValSer

CACAACAAGTTCCAGCAGTTCAACGTCGCCAACCCTGGCGTGGTCTTCAAC 612
HisAsnLysPheGlnGlnPheAsnValAlaAsnProGlyValValPheAsn

AACGGCCTGACCGACGGCGTGTCCAGGATCGGCGGGGCGCTGACCAAGAAC 663
AsnGlyLeuThrAspGlyValSerArgIleGlyGlyAlaLeuThrLysAsn

CCCAACCTGACTCGCCAGGCCTCGGCCATTCTTGCCGAAGTCACGGACACT 714
ProAsnLeuThrArgGlnAlaSerAlaIleLeuAlaGluValThrAspThr

TCGCCCAGTCGCCTGGCCGGTACGCTCGAAGTCTATGGCAAGGGCGCCGAC 765
SerProSerArgLeuAlaGlyThrLeuGluValTyrGlyLysGlyAlaAsp

CTCATCATCGCCAACCCCAACGGCATCAGCGTCAACGGCCTGAGCACGCTC 816
LeuIleIleAlaAsnProAsnGlyIleSerValAsnGlyLeuSerThrLeu

AACGCCAGCAACCTGACGCTCACGACGGGGCGTCCCAGCGTCAACGGCGGC 867
AsnAlaSerAsnLeuThrLeuThrThrGlyArgProSerValAsnGlyGly

CGCATCGGCCTTGATGTCCAACAGGGCACCGTCACGATCGAACGAGGCGGC 918
ArgIleGlyLeuAspValGlnGlnGlyThrValThrIleGluArgGlyGly

GTCAATGCCACCGGCCTGGGCTATTTCGACGTGGTGGCGCGCCTGGTCAAG 969
ValAsnAlaThrGlyLeuGlyTyrPheAspValValAlaArgLeuValLys

CTGCAGGGTGCCGTGTCGAGCAAGCAGGGCAAGCCCCTGGCCGACATCGCG 1020
LeuGlnGlyAlaValSerSerLysGlnGlyLysProLeuAlaAspIleAla

GTGGTCGCCGGCGCCAACCGGTACGACCACGCAACCCGCCGCGCCACGCCG 1071
ValValAlaGlyAlaAsnArgTyrAspHisAlaThrArgArgAlaThrPro

ATCGCCGCAGGCGCGCGCGGCGCCGCCGCGGGCGCCTACGCGATTGACGGC 1122
IleAlaAlaGlyAlaArgGlyAlaAlaAlaGlyAlaTyrAlaIleAspGly

ACGGCGGCGGGCGCCATGTACGGCAAGCACATCACGCTGGTGTCCAGCGAT 1173
ThrAlaAlaGlyAlaMetTyrGlyLysHisIleThrLeuValSerSerAsp

TCAGGCCTGGGCGTGCGCCAGCTCGGCAGCCTGTCCTCGCCATCGGCCATC 1224
SerGlyLeuGlyValArgGlnLeuGlySerLeuSerSerProSerAlaIle

ACCGTGTCGTCGCAGGGCGAAATCGCGCTGGGCGACGCCACGGTCCAGCGC 1275
ThrValSerSerGlnGlyGluIleAlaLeuGlyAspAlaThrValGlnArg

GGCCCGCTCAGCCTCAAGGGCGCGGGGGTCGTGTCGGCCGGCAAACTGGCC 1326
GlyProLeuSerLeuLysGlyAlaGlyValValSerAlaGlyLysLeuAla

TCCGGGGGGGGGGCGGTGAACGTCGCGGGCGGCGGGGCGGTGAAGATCGCG 1377
SerGlyGlyGlyAlaValAsnValAlaGlyGlyGlyAlaValLysIleAla

TCGGCCAGCAGCGTTGGAAACCTCGCGGTGCAAGGCGGCGGCAAGGTACAG 1428
SerAlaSerSerValGlyAsnLeuAlaValGlnGlyGlyGlyLysValGln

GCCACGCTGTTGAATGCCGGGGGGACGTTGCTGGTGTCGGGCCGCCAGGCC 1479
AlaThrLeuLeuAsnAlaGlyGlyThrLeuLeuValSerGlyArgGlnAla

GTCCAGCTTGGCGCGGCGAGCAGCCGTCAGGCGCTGTCCGTGAACGCGGGC 1530
ValGlnLeuGlyAlaAlaSerSerArgGlnAlaLeuSerValAsnAlaGly

GGCGCCCTCAAGGCGGACAAGCTGTCGGCGACGCGACGGGTCGACGTGGAT 1581
GlyAlaLeuLysAlaAspLysLeuSerAlaThrArgArgValAspValAsp

GGCAAGCAGGCCGTCGCGCTGGGGTCGGCCAGCAGCAATGCGCTGTCGGTG 1632
GlyLysGlnAlaValAlaLeuGlySerAlaSerSerAsnAlaLeuSerVal

CGTGCCGGCGGCGCCCTCAAGGCGGGCAAGCTGTCGGCGACGGGGCGACTG 1683
ArgAlaGlyGlyAlaLeuLysAlaGlyLysLeuSerAlaThrGlyArgLeu

GACGTGGACGGCAAGCAGGCCGTCACGCTGGGTTCGGTTGCGAGCGACGGT 1734
AspValAspGlyLysGlnAlaValThrLeuGlySerValAlaSerAspGly

GCGCTGTCGGTAAGCGCTGGCGGAAACCTGCGGGCGAACGAATTGGTCTCC 1785
AlaLeuSerValSerAlaGlyGlyAsnLeuArgAlaAsnGluLeuValSer

AGTGCCCAACTTGAGGTGCGTGGGCAGCGGGAGGTCGCGCTGGATGACGCT 1836
SerAlaGlnLeuGluValArgGlyGlnArgGluValAlaLeuAspAspAla

TCGAGCGCACGCGGCATGACCGTGGTTGCCGCAGGAGCGCTGGCGGCCCGC 1887
SerSerAlaArgGlyMetThrValValAlaAlaGlyAlaLeuAlaAlaArg

AACCTGCAGTCCAAGGGCGCCATCGGCGTACAGGGTGGAGAGGCGGTCAGC 1938
AsnLeuGlnSerLysGlyAlaIleGlyValGlnGlyGlyGluAlaValSer

GTGGCCAACGCGAACAGCGACGCGGAATTGCGCGTGCGCGGGCGCGGCCAG 1989
ValAlaAsnAlaAsnSerAspAlaGluLeuArgValArgGlyArgGlyGln

GTGGATCTGCACGACCTGAGCGCAGCGCGCGGCGCGGATATCTCCGGCGAG 2040
ValAspLeuHisAspLeuSerAlaAlaArgGlyAlaAspIleSerGlyGlu

GGGCGCGTCAATATCGGCCGTGCGCGCAGCGATAGCGATGTGAAGGTCTCC 2091
GlyArgValAsnIleGlyArgAlaArgSerAspSerAspValLysValSer

GCGCACGGCGCCTTGTCGATCGATAGCATGACGGCCCTCGGTGCGATCGGC 2142
AlaHisGlyAlaLeuSerIleAspSerMetThrAlaLeuGlyAlaIleGly

GTCCAGGCAGGCGGCAGCGTGTCGGCCAAGGATATGCGCAGCCGTGGCGCC 2193
ValGlnAlaGlyGlySerValSerAlaLysAspMetArgSerArgGlyAla

GTCACCGTCAGCGGCGGCGGCGCCGTCAACCTGGGCGATGTCCAGTCGGAT 2244
ValThrValSerGlyGlyGlyAlaValAsnLeuGlyAspValGlnSerAsp

GGGCAGGTCCGCGCCACCAGCGCGGGCGCCATGACGGTGCGAGACGTCGCG 2295
GlyGlnValArgAlaThrSerAlaGlyAlaMetThrValArgAspValAla

GCTGCCGCCGACCTTGCGCTGCAGGCGGGCGACGCGTTGCAGGCCGGGTTC 2346
AlaAlaAlaAspLeuAlaLeuGlnAlaGlyAspAlaLeuGlnAlaGlyPhe

CTGAAATCGGCCGGTGCCATGACCGTGAACGGCCGCGATGCCGTGCGACTG 2397
LeuLysSerAlaGlyAlaMetThrValAsnGlyArgAspAlaValArgLeu

GATGGCGCGCACGCGGGCGGGCAATTGCGGGTTTCCAGCGACGGGCAGGCT 2448
AspGlyAlaHisAlaGlyGlyGlnLeuArgValSerSerAspGlyGlnAla

GCGTTGGGCAGTCTCGCGGCCAAGGGCGAGCTGACGGTATCGGCCGCGCGC 2499
AlaLeuGlySerLeuAlaAlaLysGlyGluLeuThrValSerAlaAlaArg

GCGGCGACCGTGGCCGAGTTGAAGTCGCTGGACAACATCTCCGTGACGGGC 2550
AlaAlaThrValAlaGluLeuLysSerLeuAspAsnIleSerValThrGly

GGCGAACGCGTGTCGGTTCAGAGCGTCAACAGCGCGTCCAGGGTCGCCATT 2601
GlyGluArgValSerValGlnSerValAsnSerAlaSerArgValAlaIle

TCGGCGCACGGCGCGCTGGATGTAGGCAAGGTTTCCGCCAAGAGCGGTATC 2652
SerAlaHisGlyAlaLeuAspValGlyLysValSerAlaLysSerGlyIle

GGGCTCGAAGGCTGGGGCGCGGTCGGAGCGGACTCCCTCGGTTCCGACGGC 2703
GlyLeuGluGlyTrpGlyAlaValGlyAlaAspSerLeuGlySerAspGly

GCGATCAGCGTGTCCGGGCGCGATGCGGTCAGGGTCGATCAAGCCCGCAGT 2754
AlaIleSerValSerGlyArgAspAlaValArgValAspGlnAlaArgSer

CTTGCCGACATTTCGCTGGGGGCGGAAGGCGGCGCCACGCTGGGCGCGGTG 2805
LeuAlaAspIleSerLeuGlyAlaGluGlyGlyAlaThrLeuGlyAlaVal

GAGGCCGCCGGTTCGATCGACGTGCGCGGCGGATCCACGGTGGCGGCGAAC 2856
GluAlaAlaGlySerIleAspValArgGlyGlySerThrValAlaAlaAsn

TCGCTGCACGCCAATCGCGACGTTCGGGTCAGCGGCAAGGATGCGGTGCGC 2907
SerLeuHisAlaAsnArgAspValArgValSerGlyLysAspAlaValArg

GTAACGGCCGCCACCAGCGGGGGCGGTCTGCATGTGTCGAGCGGCCGCCAG 2958
ValThrAlaAlaThrSerGlyGlyGlyLeuHisValSerSerGlyArgGln

CTCGATCTGGGCGCCGTGCAGGCGCGCGGCGCGCTGGCCCTGGACGGAGGC 3009
LeuAspLeuGlyAlaValGlnAlaArgGlyAlaLeuAlaLeuAspGlyGly

GCCGGCGTGGCGCTGCAATCGGCCAAGGCTAGCGGCACGCTGCATGTGCAG 3060
AlaGlyValAlaLeuGlnSerAlaLysAlaSerGlyThrLeuHisValGln

GGCGGCGAGCACCTGGACCTGGGCACGTTGGCCGCCGTAGGGGCGGTGGAC 3111
GlyGlyGluHisLeuAspLeuGlyThrLeuAlaAlaValGlyAlaValAsp

GTCAATGGCACGGGAGACGTGCGCGTTGCGAAGCTGGTGAGCGATGCAGGC 3162
ValAsnGlyThrGlyAspValArgValAlaLysLeuValSerAspAlaGly

GCCGATCTGCAAGCGGGGCGCTCCATGACGCTGGGTATCGTCGACACGACC 3213
AlaAspLeuGlnAlaGlyArgSerMetThrLeuGlyIleValAspThrThr

GGCGATCTGCAGGCGCGCGCGCAGCAGAAGCTGGAGCTCGGGTCGGTTAAG 3264
GlyAspLeuGlnAlaArgAlaGlnGlnLysLeuGluLeuGlySerValLys

AGCGATGGCGGCCTTCAGGCGGCCGCCGGCGGGGCCCTCAGCCTGGCGGCG 3315
SerAspGlyGlyLeuGlnAlaAlaAlaGlyGlyAlaLeuSerLeuAlaAla

GCGGAAGTCGCAGGGGCGCTGGAGCTCTCGGGCCAGGGCGTCACCGTGGAC 3366
AlaGluValAlaGlyAlaLeuGluLeuSerGlyGlnGlyValThrValAsp

AGAGCCAGCGCTAGCCGGGCACGCATCGACAGCACCGGTTCGGTCGGCATC 3417
ArgAlaSerAlaSerArgAlaArgIleAspSerThrGlySerValGlyIle

GGCGCGCTGAAGGCAGGCGCTGTCGAGGCCGCCTCGCCACGGCGGGCGCGC 3468
GlyAlaLeuLysAlaGlyAlaValGluAlaAlaSerProArgArgAlaArg

CGCGCGCTGCGGCAGGATTTCTTCACGCCCGGCAGCGTGGTGGTCCGCGCC 3519
ArgAlaLeuArgGlnAspPhePheThrProGlySerValValValArgAla

CAGGGCAATGTCACGGTCGGGCGCGGCGATCCGCATCAGGGCGTGCTGGCC 3570
GlnGlyAsnValThrValGlyArgGlyAspProHisGlnGlyValLeuAla

CAGGGCGACATCATCATGGATGCGAAGGGCGGCACCTTGCTGTTGCGCAAC 3621
GlnGlyAspIleIleMetAspAlaLysGlyGlyThrLeuLeuLeuArgAsn

GATGCCTTGACCGAGAACGGGACGGTCACCATATCGGCCGATTCGGCCGTG 3672
AspAlaLeuThrGluAsnGlyThrValThrIleSerAlaAspSerAlaVal

CTCGAGCATTCCACCATCGAGAGCAAGATCAGCCAGAGCGTGCTGGCTGCC 3723
LeuGluHisSerThrIleGluSerLysIleSerGlnSerValLeuAlaAla

AAAGGGGACAAGGGCAAGCCGGCGGTGTCGGTGAAGGTCGCGAAGAAGCTG 3774
LysGlyAspLysGlyLysProAlaValSerValLysValAlaLysLysLeu

TTTCTCAATGGTACGTTGCGGGCCGTCAACGACAACAACGAAACCATGTCC 3825
PheLeuAsnGlyThrLeuArgAlaValAsnAspAsnAsnGluThrMetSer

GGGCGCCAGATCGACGTCGTGGACGGACGTCCGCAGATCACCGACGCGGTC 3876
GlyArgGlnIleAspValValAspGlyArgProGlnIleThrAspAlaVal

ACGGGCGAAGCGCGTAAGGACGAATCGGTTGTGTCCGACGCCGCGCTCGTG 3927
ThrGlyGluAlaArgLysAspGluSerValValSerAspAlaAlaLeuVal

GCCGATGGCGGTCCGATCGTGGTCGAGGCCGGCGAGCTGGTCAGCCATGCC 3978
AlaAspGlyGlyProIleValValGluAlaGlyGluLeuValSerHisAla

GGCGGTATCGGCAACGGCCGCAACAAGGAGAATGGCGCCAGCGTCACCGTG 4029
GlyGlyIleGlyAsnGlyArgAsnLysGluAsnGlyAlaSerValThrVal

CGCACGACTGGCAACCTGGTCAACAAGGGCTACATCTCGGCCGGCAAGCAG 4080
ArgThrThrGlyAsnLeuValAsnLysGlyTyrIleSerAlaGlyLysGln

GGCGTGCTCGAGGTGGGCGGCGCCTTGACGAACGAGTTCCTGGTCGGCTCG 4131
GlyValLeuGluValGlyGlyAlaLeuThrAsnGluPheLeuValGlySer

GACGGCACCCAGCGCATCGAGGCGCAGCGCATCGAGAACAGGGGCACCTTC 4182
AspGlyThrGlnArgIleGluAlaGlnArgIleGluAsnArgGlyThrPhe

CAGAGCCAGGCTCCGGCGGGCACGGCCGGCGCCCTGGTGGTCAAGGCTGCC 4233
GlnSerGlnAlaProAlaGlyThrAlaGlyAlaLeuValValLysAlaAla

GAGGCCATCGTGCACGACGGCGTCATGGCCACCAAAGGCGAGATGCAGATC 4284
GluAlaIleValHisAspGlyValMetAlaThrLysGlyGluMetGlnIle

GCCGGCAAGGGCGGCGGGTCTCCGACCGTCACCGCCGGCGCAAAGGCGACG 4335
AlaGlyLysGlyGlyGlySerProThrValThrAlaGlyAlaLysAlaThr

ACCAGCGCGAACAAGCTGAGCGTCGACGTGGCAAGCTGGGACAACGCGGGA 4386
ThrSerAlaAsnLysLeuSerValAspValAlaSerTrpAspAsnAlaGly

AGCCTGGATATCAAGAAGGGCGGCGCGCAGGTCACGGTGGCCGGGCGCTAT 4437
SerLeuAspIleLysLysGlyGlyAlaGlnValThrValAlaGlyArgTyr

GCCGAGCACGGCGAGGTTTCGATACAGGGCGATTACACCGTCTCGGCCGAC 4488
AlaGluHisGlyGluValSerIleGlnGlyAspTyrThrValSerAlaAsp

GCCATCGCGCTGGCGGCGCAGGTCACCCAGCGCGGAGGCGCCGCGAACCTG 4539
AlaIleAlaLeuAlaAlaGlnValThrGlnArgGlyGlyAlaAlaAsnLeu

ACCTCGCGGCACGACACCCGTTTCTCCAACAAGATTCGCCTGATGGGGCCG 4590
ThrSerArgHisAspThrArgPheSerAsnLysIleArgLeuMetGlyPro

TTGCAGGTCAACGCCGGCGGGCCGGTGTCCAATACCGGCAATCTGAAAGTG 4641
LeuGlnValAsnAlaGlyGlyProValSerAsnThrGlyAsnLeuLysVal

CGCGAGGGCGTGACCGTAACGGCGGCGTCGTTCGACAACGAGACCGGGGCC 4692
ArgGluGlyValThrValThrAlaAlaSerPheAspAsnGluThrGlyAla

GAGGTCATGGCCAAGAGCGCCACGCTGACGACTTCCGGGGCCGCGCGCAAC 4743
GluValMetAlaLysSerAlaThrLeuThrThrSerGlyAlaAlaArgAsn

GCGGGCAAGATGCAGGTCAAGGAGGCCGCCACGATCGTTGCCGCCAGCGTT 4794
AlaGlyLysMetGlnValLysGluAlaAlaThrIleValAlaAlaSerVal

TCCAATCCCGGCACGTTCACGGCCGGCAAGGATATCACTGTTACCTCGCGC 4845
SerAsnProGlyThrPheThrAlaGlyLysAspIleThrValThrSerArg

GGAGGATTCGATAACGAAGGCAAGATGGAGTCCAACAAGGACATCGTCATC 4896
GlyGlyPheAspAsnGluGlyLysMetGluSerAsnLysAspIleValIle

AAGACGGAACAGTTCAGCAATGGCAGGGTTCTCGACGCCAAGCATGATCTG 4947
LysThrGluGlnPheSerAsnGlyArgValLeuAspAlaLysHisAspLeu

ACGGTCACGGCGAGCGGGCAGGCGGACAACCGGGGCAGCCTGAAGGCAGGC 4998
ThrValThrAlaSerGlyGlnAlaAspAsnArgGlySerLeuLysAlaGly

CACGATTTCACGGTGCAGGCCCAGCGTATCGACAATAGCGGAACCATGGCC 5049
HisAspPheThrValGlnAlaGlnArgIleAspAsnSerGlyThrMetAla

GCCGGCCACGACGCCACGCTGAAGGCGCCGCACCTGCGCAATACGGGCCAG 5100
AlaGlyHisAspAlaThrLeuLysAlaProHisLeuArgAsnThrGlyGln

GTCGTAGCCGGGCACGACATCCATATCATCAACAGCGCCAAGCTGGAGAAC 5151
ValValAlaGlyHisAspIleHisIleIleAsnSerAlaLysLeuGluAsn

ACCGGGCGCGTGGATGCGCGCAACGACATCGCTCTGGATGTGGCGGATTTC 5202
ThrGlyArgValAspAlaArgAsnAspIleAlaLeuAspValAlaAspPhe

ACCAACACGGGATCCCTCTACGCCGAGCATGACGCGACGCTGACGCTTGCG 5253
ThrAsnThrGlySerLeuTyrAlaGluHisAspAlaThrLeuThrLeuAla

CAAGGCACGCAGCGCGATCTGGTGGTGGACCAGGATCATATCCTGCCGGTG 5304
GlnGlyThrGlnArgAspLeuValValAspGlnAspHisIleLeuProVal

GCGGAGGGGACGTTACGCGTCAAGGCCAAGTCGCTGACCACCGAAATCGAG 5355
AlaGluGlyThrLeuArgValLysAlaLysSerLeuThrThrGluIleGlu

ACCGGCAATCCCGGCAGCCTGATCGCCGAGGTGCAGGAAAATATCGACAAC 5406
ThrGlyAsnProGlySerLeuIleAlaGluValGlnGluAsnIleAspAsn

AAGCAGGCCATCGTCGTCGGCAAGGACCTGACGCTGAGTTCGGCGCACGGC 5457
LysGlnAlaIleValValGlyLysAspLeuThrLeuSerSerAlaHisGly

AACGTGGCCAACGAAGCGAACGCGCTGCTGTGGGCCGCCGGGGAGCTGACC 5508
AsnValAlaAsnGluAlaAsnAlaLeuLeuTrpAlaAlaGlyGluLeuThr

GTCAAGGCGCAGAACATCACCAATAAACGGGCCGCGCTGATCGAGGCGGGC 5559
ValLysAlaGlnAsnIleThrAsnLysArgAlaAlaLeuIleGluAlaGly

GGCAACGCCCGGCTGACGGCGGCCGTTGCCTTGCTCAACAAGCTGGGCCGC 5610
GlyAsnAlaArgLeuThrAlaAlaValAlaLeuLeuAsnLysLeuGlyArg

ATTCGCGCGGGCGAGGACATGCACCTGGATGCGCCGCGCATCGAGAACACC 5661
IleArgAlaGlyGluAspMetHisLeuAspAlaProArgIleGluAsnThr

GCGAAACTGAGCGGCGAGGTGCAACGCAAAGGCGTGCAGGACGTCGGGGGA 5712
AlaLysLeuSerGlyGluValGlnArgLysGlyValGlnAspValGlyGly

GGCGAGCACGGCCGCTGGAGCGGTATCGGCTATGTCAACTACTGGTTGCGC 5763
GlyGluHisGlyArgTrpSerGlyIleGlyTyrValAsnTyrTrpLeuArg

GCCGGCAATGGGAAGAAGGCGGGAACCATCGCCGCGCCGTGGTATGGCGGT 5814
AlaGlyAsnGlyLysLysAlaGlyThrIleAlaAlaProTrpTyrGlyGly

GATCTGACGGCGGAGCAGTCGCTCATCGAGGTCGGCAAGGATCTCTATCTG 5865
AspLeuThrAlaGluGlnSerLeuIleGluValGlyLysAspLeuTyrLeu

AATGCCGGAGCGCGCAAGGACGAACATCGCCATCTGCTCAATGAAGGCGTG 5916
AsnAlaGlyAlaArgLysAspGluHisArgHisLeuLeuAsnGluGlyVal

ATCCAGGCGGGCGGCCATGGCCACATCGGCGGCGACGTGGACAACCGGTCG 5967
IleGlnAlaGlyGlyHisGlyHisIleGlyGlyAspValAspAsnArgSer

GTGGTGCGCACCGTGTCCGCCATGGAGTATTTCAAGACGCCTCTTCCGGTG 6018
ValValArgThrValSerAlaMetGluTyrPheLysThrProLeuProVal

AGCCTGACTGCCCTGGACAATCGTGCCGGCTTGTCTCCGGCGACCTGGAAC 6069
SerLeuThrAlaLeuAspAsnArgAlaGlyLeuSerProAlaThrTrpAsn

TTCCAGTCCACGTATGAACTCCTGGATTATCTGCTGGACCAGAATCGCTAC 6120
PheGlnSerThrTyrGluLeuLeuAspTyrLeuLeuAspGlnAsnArgTyr

GAGTACATTTGGGGGCTGTATCCGACCTACACCGAATGGTCGGTGAATACG 6171
GluTyrIleTrpGlyLeuTyrProThrTyrThrGluTrpSerValAsnThr

CTGAAGAACCTCGACCTGGGCTACCAGGCCAAGCCGGCTCCCACTGCGCCG 6222
LeuLysAsnLeuAspLeuGlyTyrGlnAlaLysProAlaProThrAlaPro

CCGATGCCCAAGGCTCCCGAACTCGACCTGCGTGGCCATACGCTGGAGTCG 6273
ProMetProLysAlaProGluLeuAspLeuArgGlyHisThrLeuGluSer

GCCGAAGGCCGGAAGATCTTTGGCGAGTACAAGAAGCTGCAAGGCGAGTAC 6324
AlaGluGlyArgLysIlePheGlyGluTyrLysLysLeuGlnGlyGluTyr

GAGAAGGCGAAGATGGCCGTCCAGGCCGTGGAGGCTTACGGCGAGGCTACT 6375
GluLysAlaLysMetAlaValGlnAlaValGluAlaTyrGlyGluAlaThr

CGGCGCGTCCATGATCAGCTGGGCCAACGTTATGGTAAGGCCCTGGGCGGC 6426
ArgArgValHisAspGlnLeuGlyGlnArgTyrGlyLysAlaLeuGlyGly

ATGGATGCCGAGACCAAGGAGGTCGACGGCATCATCCAGGAGTTCGCCGCG 6477
MetAspAlaGluThrLysGluValAspGlyIleIleGlnGluPheAlaAla

GATCTGCGAACGGTCTATGCGAAGCAGGCCGACCAGGCGACCATCGACGCA 6528
AspLeuArgThrValTyrAlaLysGlnAlaAspGlnAlaThrIleAspAla

GAGACGGACAAGGTCGCCCAGCGCTACAAGTCGCAGATCGACGCGGTGCGG 6579
GluThrAspLysValAlaGlnArgTyrLysSerGlnIleAspAlaValArg

CTGCAGGCGATCCAGCCTGGCCGGGTCACGCTGGCCAAGGCGCTGTCGGCG 6630
LeuGlnAlaIleGlnProGlyArgValThrLeuAlaLysAlaLeuSerAla

GCGCTGGGCGCCGACTGGCGCGCGCTGGGTCACTCCCAATTGATGCAGCGC 6681
AlaLeuGlyAlaAspTrpArgAlaLeuGlyHisSerGlnLeuMetGlnArg

TGGAAGGATTTCAAGGCGGGCAAGCGCGGCGCGGAAATCGCGTTCTATCCC 6732
TrpLysAspPheLysAlaGlyLysArgGlyAlaGluIleAlaPheTyrPro

AAGGAACAAACCGTGCTGGCCGCCGGCGCCGGTTTGACCCTGTCCAACGGG 6783
LysGluGlnThrValLeuAlaAlaGlyAlaGlyLeuThrLeuSerAsnGly

GCGATCCACAACGGCGAGAACGCCGCGCAGAATCGCGGCCGGCCGGAAGGC 6834
AlaIleHisAsnGlyGluAsnAlaAlaGlnAsnArgGlyArgProGluGly

CTGAAAATCGGCGCACATTCGGCGACTTCGGTGAGCGGCTCGTTCGACGCC 6885
LeuLysIleGlyAlaHisSerAlaThrSerValSerGlySerPheAspAla

TTGCGCGACGTGGGGCTGGAAAAGCGGCTGGATATCGACGATGCGCTGGCT 6936
LeuArgAspValGlyLeuGluLysArgLeuAspIleAspAspAlaLeuAla

GCCGTGCTCGTGAATCCGCATATTTTCACGCGGATCGGGGCGGCTCAGACA 6987
AlaValLeuValAsnProHisIlePheThrArgIleGlyAlaAlaGlnThr

TCCCTTGCCGACGGCGCCGCCGGGCCGGCGCTGGCGCGCCAGGCCAGGCAA 7038
SerLeuAlaAspGlyAlaAlaGlyProAlaLeuAlaArgGlnAlaArgGln

GCGCCGGAGACCGACGGCATGGTGGATGCGCGAGGGCTGGGCAGCGCCGAT 7089
AlaProGluThrAspGlyMetValAspAlaArgGlyLeuGlySerAlaAsp

GCGCTCGCTTCCCTGGCCAGCTTGGACGCGGCGCAAGGGCTGGAGGTATCC 7140
AlaLeuAlaSerLeuAlaSerLeuAspAlaAlaGlnGlyLeuGluValSer

GGCAGGCGCAATGCGCAGGTGGCCGACGCCGGGCTCGCCGGGCCGAGCGCC 7191
GlyArgArgAsnAlaGlnValAlaAspAlaGlyLeuAlaGlyProSerAla

GTCGCGGCGCCGGCCGTCGGGGCGGCCGATGTCGGCGTGGAGCCTGTCACG 7242
ValAlaAlaProAlaValGlyAlaAlaAspValGlyValGluProValThr

GGGGACCAGGTCGACCAGCCTGTCGTGGCGGTCGGGCTCGAGCAGCCTGTC 7293
GlyAspGlnValAspGlnProValValAlaValGlyLeuGluGlnProVal

GCGACGGTCCGGGTCGCGCCGCCAGCCGTCGCGTTGCCGCGGCCGCTGTTC 7344
AlaThrValArgValAlaProProAlaValAlaLeuProArgProLeuPhe

GAAACCCGCATCAAGTTTATCGACCAGAGCAAATTCTACGGCTCGCGTTAT 7395
GluThrArgIleLysPheIleAspGlnSerLysPheTyrGlySerArgTyr

TTCTTCGAGCAGATCGGCTACAAGCCCGATCGCGCCGCGCGGGTGGCGGGC 7446
PhePheGluGlnIleGlyTyrLysProAspArgAlaAlaArgValAlaGly

GACAACTATTTCGATACCACGCTGGTGCGCGAGCAGGTGCGGCGCGCCCTG 7497
AspAsnTyrPheAspThrThrLeuValArgGluGlnValArgArgAlaLeu

GGCGGCTATGAAAGCCGCCTGCCCGTGCGCGGTGTCGCGTTGGTGGCCAAG 7548
GlyGlyTyrGluSerArgLeuProValArgGlyValAlaLeuValAlaLys

CTGATGGATTCGGCCGGGACGGTCGGCAAGGCGCTGGGCCTGAAGGTGGGT 7599
LeuMetAspSerAlaGlyThrValGlyLysAlaLeuGlyLeuLysValGly

GTCGCGCCGACCGCGCAGCAGCTCAAGCAGGCCGACCGCGATTTCGTCTGG 7650
ValAlaProThrAlaGlnGlnLeuLysGlnAlaAspArgAspPheValTrp

TACGTGGATACCGTGATCGACGGCCAGAAGGTTCTCGCTCCCCGGCTGTAC 7701
TyrValAspThrValIleAspGlyGlnLysValLeuAlaProArgLeuTyr

CTGACCGAGGCGACGCGCCAGGGCATCACGGATCAGTACGCCGGCGGCGGG 7752
LeuThrGluAlaThrArgGlnGlyIleThrAspGlnTyrAlaGlyGlyGly

GCGCTGATTGCCTCCGGCGGCGACGTAACTGTCAATACGGACGGCCATGAC 7803
AlaLeuIleAlaSerGlyGlyAspValThrValAsnThrAspGlyHisAsp

GTCAGTTCGGTCAACGGGCTGATCCAGGGCAGGAGCGTCAAGGTGGACGCG 7854
ValSerSerValAsnGlyLeuIleGlnGlyArgSerValLysValAspAla

GGCAAGGGCAAGGTCGTGGTGGCCGACAGCAAGGGGGCGGGCGGCGGCATC 7905
GlyLysGlyLysValValValAlaAspSerLysGlyAlaGlyGlyGlyIle

GAGGCCGATGACGAGGTCGACGTCTCAGGCCGGGATATCGGCATCGAGGGC 7956
GluAlaAspAspGluValAspValSerGlyArgAspIleGlyIleGluGly

GGCAAGCTGCGCGGCAAGGATGTCAGGCTCAAGGCCGACACGGTCAAGGTC 8007
GlyLysLeuArgGlyLysAspValArgLeuLysAlaAspThrValLysVal

GCGACCTCGATGCGTTACGACGACAAGGGCAGGCTGGCGGCGCGCGGCGAC 8058
AlaThrSerMetArgTyrAspAspLysGlyArgLeuAlaAlaArgGlyAsp

GGCGCCCTGGATGCGCAAGGCGGCCAGCTGCATATCGAGGCCAAGCGCCTG 8109
GlyAlaLeuAspAlaGlnGlyGlyGlnLeuHisIleGluAlaLysArgLeu

GAGACGGCCGGCGCGACGCTCAAGGGCGGCAAGGTGAAGCTGGATGTCGAT 8160
GluThrAlaGlyAlaThrLeuLysGlyGlyLysValLysLeuAspValAsp

GACGTCAAGTTGGGCGGCGTGTACGAGGCGGGGTCCAGCTACGAGAACAAG 8211
AspValLysLeuGlyGlyValTyrGluAlaGlySerSerTyrGluAsnLys

AGCTCGACGCCGCTGGGCAGCCTGTTCGCCATCCTGTCGTCGACGACGGAA 8262
SerSerThrProLeuGlySerLeuPheAlaIleLeuSerSerThrThrGlu

ACCAACCAGTCGGCACACGCGAACCATTACGGTACGCGCATCGAAGCCGGT 8313
ThrAsnGlnSerAlaHisAlaAsnHisTyrGlyThrArgIleGluAlaGly

ACGCTGGAAGGAAAGATGCAGAACCTGGAGATCGAAGGCGGTTCGGTCGAT 8364
ThrLeuGluGlyLysMetGlnAsnLeuGluIleGluGlyGlySerValAsp

GCCGCGCATACGGACCTGTCCGTGGCCCGCGACGCGAGGTTCAAGGCCGCC 8415
AlaAlaHisThrAspLeuSerValAlaArgAspAlaArgPheLysAlaAla

GCGGATTTCGCGCACGCCGAGCATGAGAAGGATGTGCGCCAACTGTCCCTG 8466
AlaAspPheAlaHisAlaGluHisGluLysAspValArgGlnLeuSerLeu

GGTGCCAAGGTGGGGGCGGGCGGCTACGAGGCGGGCTTCAGCCTGGGCAGC 8517
GlyAlaLysValGlyAlaGlyGlyTyrGluAlaGlyPheSerLeuGlySer

GAAAGCGGTCTGGAAGCGCACGCCGGCCGCGGTATGACCGCGGGCGCTGAA 8568
GluSerGlyLeuGluAlaHisAlaGlyArgGlyMetThrAlaGlyAlaGlu

GTCAAGGTAGGTTATCGGGCATCGCACGAACAGTCCTCGGAAACCGAAAAG 8619
ValLysValGlyTyrArgAlaSerHisGluGlnSerSerGluThrGluLys

TCCTATCGCAACGCGAACCTCAATTTCGGTGGAGGCTCCGTCGAGGCTGGC 8670
SerTyrArgAsnAlaAsnLeuAsnPheGlyGlyGlySerValGluAlaGly

AATGTCCTGGATATCGGCGGCGCCGACATCAACCGGAACCGGTACGGCGGC 8721
AsnValLeuAspIleGlyGlyAlaAspIleAsnArgAsnArgTyrGlyGly

GCCGCGAAGGGGAACGCCGGGACCGAGGAGGCCTTGCGCATGCGCGCCAAG 8772
AlaAlaLysGlyAsnAlaGlyThrGluGluAlaLeuArgMetArgAlaLys

AAGGTCGAGTCCACCAAGTACGTCAGCGAGCAGACGAGCCAGAGCTCCGGC 8823
LysValGluSerThrLysTyrValSerGluGlnThrSerGlnSerSerGly

TGGAGCGTGGAGGTGGCATCGACGGCCAGTGCCCGTTCCAGCCTGCTGACG 8874
TrpSerValGluValAlaSerThrAlaSerAlaArgSerSerLeuLeuThr

GCCGCCACGCGCCTGGGCGACAGCGTGGCGCAGAATGTCGAGGACGGCCGC 8925
AlaAlaThrArgLeuGlyAspSerValAlaGlnAsnValGluAspGlyArg

GAGATCCGCGGCGAGCTGATGGCTGCGCAAGTCGCCGCGGAGGCCACGCAA 8976
GluIleArgGlyGluLeuMetAlaAlaGlnValAlaAlaGluAlaThrGln

CTGGTAACCGCCGACACGGCGGCGGTGGCACTGAGTGCCGGAATCAGCGCC 9027
LeuValThrAlaAspThrAlaAlaValAlaLeuSerAlaGlyIleSerAla

GACTTCGACAGCAGCCACAGCCGCTCCACCTCGCAGAATACCCAATATCTG 9078
AspPheAspSerSerHisSerArgSerThrSerGlnAsnThrGlnTyrLeu

GGCGGAAACTTGTCCATCGAGGCCACCGAGGGCGATGCGACGCTGGTGGGC 9129
GlyGlyAsnLeuSerIleGluAlaThrGluGlyAspAlaThrLeuValGly

GCGAAGTTCGGCGGTGGCGACCAGGTCAGCTTGAAGGCAGCGAAGAGCGTG 9180
AlaLysPheGlyGlyGlyAspGlnValSerLeuLysAlaAlaLysSerVal

AACCTCATGGCGGCCGAATCGACCTTCGAATCGTACTCGGAGAGCCACAAC 9231
AsnLeuMetAlaAlaGluSerThrPheGluSerTyrSerGluSerHisAsn

TTCCACGCCTCCGCCGACGCGAACCTTGGCGCCAACGCCGTGCAGGGCGCC 9282
PheHisAlaSerAlaAspAlaAsnLeuGlyAlaAsnAlaValGlnGlyAla

GTTGGCCTGGGGTTGACTGCGGGTATGGGGACGTCGCATCAGATTACCAAC 9333
ValGlyLeuGlyLeuThrAlaGlyMetGlyThrSerHisGlnIleThrAsn

GAAACCGGCAAGACCTATGCCGGAACCTCGGTGGATGCGGCGAACGTGTCG 9384
GluThrGlyLysThrTyrAlaGlyThrSerValAspAlaAlaAsnValSer

ATCGATGCAGGCAAGGATCTGAACCTTTCCGGGTCCCGCGTGCGGGGTAAG 9435
IleAspAlaGlyLysAspLeuAsnLeuSerGlySerArgValArgGlyLys

CATGTTGTCCTGGATGTCGAGGGCGATATCAATGCGACCAGCAAGCAGGAT 9486
HisValValLeuAspValGluGlyAspIleAsnAlaThrSerLysGlnAsp

GAACGCAACTACAACTCCAGCGGTGGCGGTTGGGACGCCTCGGCAGGGGTG 9537
GluArgAsnTyrAsnSerSerGlyGlyGlyTrpAspAlaSerAlaGlyVal

GCGATTCAGAACCGCACGTTGGTTGCGCCCGTGGGGTCTGCCGGCTTCAAT 9588
AlaIleGlnAsnArgThrLeuValAlaProValGlySerAlaGlyPheAsn

TTCAATACGGAACACGACAATTCGCGCCTGACCAATGACGGGGCGGCGGGT 9639
PheAsnThrGluHisAspAsnSerArgLeuThrAsnAspGlyAlaAlaGly

GTCGTTGCCAGCGACGGGTTGACGGGCCATGTGAAAGGCGACGCCAACCTG 9690
ValValAlaSerAspGlyLeuThrGlyHisValLysGlyAspAlaAsnLeu

ACCGGCGCGACCATTGCCGATTTGTCGGGCAAGGGCAATCTCAAGGTCGAC 9741
ThrGlyAlaThrIleAlaAspLeuSerGlyLysGlyAsnLeuLysValAsp

GGCGCGGTCAACGCGCAGAACCTGAAAGACTACCGCGACAAGGACGGCGGC 9792
GlyAlaValAsnAlaGlnAsnLeuLysAspTyrArgAspLysAspGlyGly

AGCGGCGGCCTGAACGTGGGCATCTCGTCGACCACGCTGGCGCCCACCGTG 9843
SerGlyGlyLeuAsnValGlyIleSerSerThrThrLeuAlaProThrVal

GGCGTGGCGTTCGGCAGGGTGGCCGGAGAGGATTATCAGGCCGAGCAGCGC 9894
GlyValAlaPheGlyArgValAlaGlyGluAspTyrGlnAlaGluGlnArg

GCCACGATTGACGTCGGTCAAACCAAGGATCCCGCGCGCCTGCAGGTCGGC 9945
AlaThrIleAspValGlyGlnThrLysAspProAlaArgLeuGlnValGly

GGCGGCGTCAAGGGTACCCTCAATCAGGACGCCGCGCAGGCCACGGTCGTT 9996
GlyGlyValLysGlyThrLeuAsnGlnAspAlaAlaGlnAlaThrValVal

CAGCGCAACAAGCACTGGGCCGGAGGCGGGTCGGAATTCTCGGTGGCTGGC 10047
GlnArgAsnLysHisTrpAlaGlyGlyGlySerGluPheSerValAlaGly

AAGTCACTGAAGAAGAAGAACCAGGTCCGCCCGGTGGAGACGCCGACGCCG 10098
LysSerLeuLysLysLysAsnGlnValArgProValGluThrProThrPro

GATGTCGTGGATGGACCGCCTAGCCGTCCCACCACGCCGCCCGCGTCGCCG 10149
AspValValAspGlyProProSerArgProThrThrProProAlaSerPro

CAGCCGATCCGCGCGACGGTCGAGGTCAGTTCGCCGCCGCCGGTGTCCGTG 10200
GlnProIleArgAlaThrValGluValSerSerProProProValSerVal

GCCACGGTCGAAGTCGTGCCGCGGCCGAAGGTCGAAACCGGCTCAGCCGCT 10251
AlaThrValGluValValProArgProLysValGluThrGlySerAlaAla

TCCGCCTCGGCCGGTGGCGCCCAGGTCGTGCCGGTGACGCCTCCCAAGGTG 10302
SerAlaSerAlaGlyGlyAlaGlnValValProValThrProProLysVal

GAGGTCGCCAAGGTGGAGGTCGCCAAGGTGGAAGTCGTGCCGCGGCCGAAG 10353
GluValAlaLysValGluValAlaLysValGluValValProArgProLys

GTTGAAACGGCTCAGCCGCTTCCGCCCCGGCCGGTGGTGGCCGAGAAGGTG 10404
ValGluThrAlaGlnProLeuProProArgProValValAlaGluLysVal

ACGACGCCGGCGGTCCAGCCCCAGCTTGCCAAGGTGGAGACGGTGCAGCCG 10455
ThrThrProAlaValGlnProGlnLeuAlaLysValGluThrValGlnPro

GTGAAGCCCGAAACCACCAAGCCGTTGCCCAAGCCGCTGCCGGTGGCGAAG 10506
ValLysProGluThrThrLysProLeuProLysProLeuProValAlaLys

GTGACGAAAGCGCCGCCGCCGGTTGTGGAGACCGCCCAGCCGCTGCCGCCG 10557
ValThrLysAlaProProProValValGluThrAlaGlnProLeuProPro

GTCAAGCCACAGAAGGCGACCCCCGGCCCCGTGGCTGAGGTGGGCAAGGCT 10608
ValLysProGlnLysAlaThrProGlyProValAlaGluValGlyLysAla

ACGGTCACGACGGTGCAGGTGCAGAGTGCGCCGCCCAAGCCGGCCCCGGTG 10659
ThrValThrThrValGlnValGlnSerAlaProProLysProAlaProVal

GCCAAGCAGCCCGCGCCTGCACCGAAGCCCAAGCCCAAGCCCAAGCCCAAG 10710
AlaLysGlnProAlaProAlaProLysProLysProLysProLysProLys

GCCGAGCGTCCGAAGCCGGGCAAAACGACGCCCTTGAGCGGGCGCCACGTG 10761
AlaGluArgProLysProGlyLysThrThrProLeuSerGlyArgHisVal

GTGCAACAGCAGGTGCAGGTCTTGCAGCGGCAAGCGAGTGACATCAACAAC 10812
ValGlnGlnGlnValGlnValLeuGlnArgGlnAlaSerAspIleAsnAsn

ACCAAGAGCCTGCCTGGCGGGAAGCTGCCCAAGCCGGTCACCGTGAAGCTG 10863
ThrLysSerLeuProGlyGlyLysLeuProLysProValThrValLysLeu

ACCGACGAGAACGGCAAGCCGCAGACGTATACGATCAACCGGCGCGAGGAT 10914
ThrAspGluAsnGlyLysProGlnThrTyrThrIleAsnArgArgGluAsp

CTGATGAAGCTCAACGGCAAGGTGCTGTCCACCAAGACGACACTGGGCCTG 10965
LeuMetLysLeuAsnGlyLysValLeuSerThrLysThrThrLeuGlyLeu

GAGCAGACCTTCCGCCTGCGGTCGAGGATATCGGCGGCAAGAACTACCGGG 11016
GluGlnThrPheArgLeuArgSerArgIleSerAlaAlaArgThrThrGly

TCTTCTATGAAACCAACAAATAGGTAGTCGCGGCCTGCCGCGGCTCGGCGC 11067
SerSerMetLysProThrAsnArg

ATGGGGATTCGCAGGGTTCTCATGCGCCGGCCAATGCCGGATAGCGGTGCA 11118

ATTGCCGACCATTTCGCGCACCGCGCTCAAGGACGTAGGGTCGACGGCAGG 11169

CGGGACAGTTTTTGACGTGAAACTGACCGAGTGTCCGCAGGCATTGAATGG 11220

TCAGCAAGTGGGATTGTTCTTCGAATCTGGTGGCACGGTTGACTATACGTC 11271

GGGAAACCTGTTTGCGTATCGGGCCGATAGTCAGGGCGTCGAACAGGCTAC 11322

CGCAGAGCGAAAGCCGACAACGTGCAAGCCAATCTGGATGGTTCCGCTATT 11373

CATTTGGGCCGCAACAAGGGTGCGCAGGCTGCTCAGACGTTTCTGGTATCG 11424

CAGACGGCTGGGTCGTCGACGTACGGGGCGACCCTGCGCTATCTGGCATGC 11475

TACATCCGTTCGGGCGCTGGTTCCATTGTTGCGGGGAATCTCCGCAGTCAG 11526

GTGGGGTTCTCCGTGATGTATCCGTAGCCCGTGAAAGAGGGGTCACCCACT 11577

GCGGGGGGCCCCGGTACGGGATGGTCGGCTTGTCACGAGATTCTTGTTTTC 11628

CATTTCTTTCTTTTCACTCGGTCGCAGCGCCGGCTTGATGCATGCAAAGCA 11679

TCGATAGCTACGAACGGCCGCGATTCTTGAATCATGAATACATACGCTTGT 11730

GACGGGGCGCTCGCGAGAGCCGGCCCCAGGGATGGTTTACGCCTGCATTTA 11781

CGGTAAAGCGGCAAGGCGGCATGGCGCGCTGGCGGCGGCTGGGCGTCGCGG 11832

CGCTGGGCCATGCTGGCGAGCCTGGCGCCGGCCGCnCGGGCAGCTyGTnAT 11883

The relative GC content of the FHA ORF is 67.5%. Examination of this nucleotide sequence for transcriptional start signals indicates possible -35 and -10 consensus regions, TGGTTTGAC and TATAAAT, separated by 23 base pairs, located 174 and 142 bp upstream of the beginning of the ORF, with transcriptional initiation beginning apparently to 30 to 75 bp from the initiation condon. A possible ribosomal binding site, GAGG, occurs 90 bp upstream of the ORF. Another possible ribosomal binding site, CTGG, occurs 11 bp in front of the third ATG. Further analysis of the nucleotide sequence reveals a region of alternating direct repeats of the pattern, ABABA, located between 1468 and 1746 bp from the left hand EcoRI site. Similar repeats are found in the predicted amino acid sequence corresponding to this same region.

### Predicted Peptide Sequence

The predicted amino acid sequence of the FHA ORF is 3597 residues long, with a calculated MW of 368 kDa. This is substantially larger than published measured values. The composition of this sequence is alanine and glycine rich (27.0%) and is nearly identical to a previously published chemical analysis of the FHA amino acid composition (Sato et al., 1983, supra). The computed isoelectric point of the entire polypeptide is 6.79.

The concentration of charged residues in the FHA polypeptide chain is highest between positions 2000 and 2700. Hydrophobicity is highest in the N-terminal 300 residues and again at specific locations near residues 1800-2000 and 2400-2500. There is a highly predicted transmembrane helix between amino acid positions 44 and 69 with its transmembrane segment between residues 52 and 69.

One interesting feature of the predicted amino acid polypeptide is the sequence RRARR located at amino acid position 1069. This highly arginine rich sequence is a likely site for trypsin-like proteolytic cleavage. N-terminal amino acid sequence determinations of several of the SDS-PAGE FHA peptide bands by other workers confirms that cleavage, in fact, occurs at this location. Analysis of the resultant two parts of the FHA peptide sequence demonstrates striking differences in chemical properties: The N-terminal 98kDa fragment is highly basic with a positive hydropathy score, whereas the C- terminal 140 kDa portion is a negatively charged acidic polypeptide which has a more hydrophilic overall composition. Polypeptides of these two sizes are dominant species on FHA Western immunoblots.

### Cell Recognition Site

Located at amino acid position 1097 and again at position 2599 is the tripeptide sequence RGD. This sequence is known as a "cell recognition site" for the interaction of fibronectin and other eukaryotic extracellular matrix proteins with the integrin receptor family on a variety of eukaryotic cell surfaces (Pierschbacher and Ruoslahti, Proc. Natl. Acad. Sci. USA (1984) 81:5985-5988, Ruoslahti and Pierschbacher, Science (1987) 238:491-497). Secondary structure analysis of the polypeptide sequence adjacent to these two FHA RGD sites reveals that the first of these is highly predicted to be surface exposed, hydrophilic, and antigenic. Comparison of the FHA peptide sequence adjacent to this RGD site and the sequence surrounding the RGD in fibronectin shows identity at 7 of the 9 residues. Cleavage at the RRARR processing site would leave this first RGD sequence close to the N terminius of the 214 kDa polypeptide product.

### In vitro Cell Adherence

The role of several virulence factors in mediating adherence of B. pertussis to Chinese Hamster Ovary cells was evaluated. Table 3 indicates the findings:

**Table 3**

| ADHERENCE OF B. pertussis STRAINS TO CHO CELLS | | | | |
|---|---|---|---|---|
| | Mean adherent bacteria per CHO cell ± SD (95% | | | |
| Strain | Fha | Fim2 | Fim3confidence interval)% Wt | |
| BP536 (vir⁺) | + | + | -363±111 (243-483) | 100 |
| BP537 (vir⁻) | - | - | -2.55±2.8 (0.71-4.39) | 0.7 |
| BP101 (fhaBΔ101) | - | + | -10.8±5.2 (7.67-13.9) | 3.0 |
| BP-B52 (fim2B52, fim3::Km) | + | - | -317±158 (146-488) | 87.3 |
| BP353 (fhaA::Tn5) ∓ | | - | -23.4±13.8 (13.3-33.5) | 6.4 |
| BP-TOX6 (ptxΔ6) | + | + | -405±102 (303-507) | 112 |

The results described in the above section demonstrate that the gene encoding filamentous hemagglutinin of B. pertussis and the expressed gene product are now available in intact and modified forms, for use in diagnosis, prophylaxis and therapy of pertussis. Of particular interest is the use of the gene to prepare vaccines, where the protein may be used by itself, as a fragment, as the intact expression product of the gene or the physiologically active fragment thereof, or in combination with other pertussis proteins, particularly with modified pertussis toxin, or with proteins of other pathogens. The subject gene may be used to enhance the amount of the filamentous hemagglutinin present in a live or dead B. pertussis organism or to provide for the presence of the subject proteins in other organisms, where immune response to more than one antigen is desired.

## Claims

1. A nucleic acid sequence of less than 15kb comprising the 10,788 kb *B.pertussis fha*B gene free from the *fha*A gene.

2. A nucleic acid sequence of less than 15kb comprising a sequence of nucleotides encoding the *B.pertussis* FHA protein of 3597 amino acids.

3. A nucleic acid sequence according to claim 1 or 2 wherein the sequence comprises nucleotides 253 to 11,040 as set out in the accompanying description.

4. A fragment of a nucleic acid sequence of any preceding claim encoding an approximately 230kDa portion of the FHA protein, said portion being the N-proximal portion of said protein.

5. A fragment of a nucleic acid sequence of any one of claims 1 to 3 which encodes a peptide of at least 12 amino acids in length, said fragment being a fragment of the open reading frame of the *FhaB* gene which encodes the peptide encoded by the sequence of claim 3, wherein said nucleotides are characterised by a motif selected from the group:
a) nucleotides encoding the RGD cell recognition sites at positions 1097 or 2599 of the FHA polypeptide;
b) nucleotides encoding the RRARR proteolytic site at position 1069 of the FHA polypeptide;
c) nucleotides encoding direct repeats located between nucleotides 1468 and 1746 of the *FhaB* gene;
d) nucleotides encoding the motif EARKDE of positions 1211 to 1216 of the FHA polypeptide;
e) nucleotides encoding the motif SKQDER at positions 3075 to 3080 of the FHA polypeptide;
f) nucleotides encoding the (PK)₅ repeat which repeat begins at residue 3477 of the FHA polypeptide; and
g) nucleotides encoding the sequence VEVVPRKVET starting at positions 3319 and 3350 of the FHA polypeptide.

6. A fragment according to claim 5 which comprises at least 100 base pairs.

7. A fragment according to claim 5 or 6 which encodes the RGD site at position 1097.

8. An isolated polypeptide fragment of FHA encoded by the open reading frame of the nucleic acid fragment of any one of claims 5 to 7.

9. A fragment of a nucleic acid sequence of any one of claims 1 to 3 which encodes a a peptide of at least 12 amino acids in length, said peptide consisting essentially of the amino acid sequence E-A-R-K-D-E together with its adjoining sequences up to 100 amino acids in either direction.

10. An isolated polypeptide of at least 12 amino acids in length which is a fragment of the *B.pertussis* FHA protein of 3597 amino acids and which consists essentially of the amino acid sequence E-A-R-K-D-E together with its adjoining sequences up to 100 amino acids in either direction.

11. An expression construct which comprises the nucleic acid of any one of claims 1 to 3 or the fragment of any one of claims 4 to 7 or 9, said nucleic acid or fragment being operably linked to a transcription initiation region and a transcription termination region.

12. A prokaryotic cell transformed with:
(a) a nucleic acid according to any one of claims 1 to 3 or fragment thereof according to any one of claims 4 to 7 or 9, said nucleic acid or fragment being joined to a DNA sequence other than a nucleic acid sequence from *B.pertussis;* or
(b) an expression construct according to claim 11.

13. A prokaryotic cell according to claim 12 which is *B.pertussis.*

14. A method for producing a polypeptide encoded by a nucleic acid according to any one of claims 1 to 3 or fragment thereof according to any one of claims 4 to 7 or 9, said method comprising culturing a host cell according to claim 12 or 13 under conditions which result in the expression of the polypeptide; and recovering the polypeptide.

15. A vaccine comprising a polypeptide recognized by antibodies to the filamentous hemagglutinin of *B.pertussis* obtained or obtainable by the method of claim 14.

16. Use of a polypeptide obtained or obtainable by the method of claim 14 in the manufacture of a medicament for use in a method of treatment or diagnosis of the human or animal body.

## Patentansprüche

1. Nukleinsäuresequenz mit weniger als 15 kb, die das 10.788 kb B.pertussis-fhaB-Gen frei vom fhaA-Gen umfaßt.

2. Nukleinsäuresequenz mit weniger als 15 kb, die eine Sequenz von Nukleotiden umfaßt, die für das B.pertussis-FHA-Protein mit 3597 Aminosäuren kodiert.

3. Nukleinsäuresequenz nach Anspruch 1 oder 2, worin die Sequenz die Nukleotide 253 bis 11.040 umfaßt, wie in der beiliegenden Beschreibung dargelegt.

4. Fragment einer Nukleinsäuresequenz nach einem der vorangegangenen Ansprüche, das für einen Abschnitt mit etwa 230 kDa des FHA-Proteins kodiert, wobei der Abschnitt der N-proximale Abschnitt des Proteins ist.

5. Fragment einer Nukleinsäuresequenz nach einem der Ansprüche 1 bis 3, welches für ein Peptid mit einer Länge von zurnindest 12 Aminosäuren kodiert, wobei das Fragment ein Fragment des offenen Leserahmens des FhaB-Gens ist, das für das Peptid kodiert, für das die Sequenz nach Anspruch 3 kodiert, worin die Nukleotide durch ein Motiv gekennzeichnet sind, das aus der folgenden Gruppe ausgewählt ist:
a) Nukleotide, die für die RGD-Zellerkennungsstellen an den Positionen 1097 oder 2599 des FHA-Polypeptids kodieren;
b) Nukleotide, die für das proteolytische RRARR-Stelle an Position 1069 des FHA-Polypeptids kodieren;
c) Nukleotide, die für direkte Wiederholungen kodieren, die sich zwischen den Nukleotiden 1468 und 1746 des FhaB-Gens befinden;
d) Nukleotide, die für das Motiv EARKDE der Positionen 1211 bis 1216 des FHA-Polypeptids kodieren;
e) Nukleotide, die für das Motiv SKQDER an den Positionen 3075 bis 3080 des FHA-Polypeptids kodieren;
f) Nukleotide, die für die (PK)₅-Wiederholung kodieren, welche Wiederholung an Rest 3477 des FHA-Polypeptids beginnt; und
g) Nukleotide, die für die Sequenz VEVVPRKVET kodieren, die an den Positionen 3319 und 3350 des FHA-Polypeptids beginnt.

6. Fragment nach Anspruch 5, das zumindest 100 Basenpaare umfaßt.

7. Fragment nach Anspruch 5 oder 6, das für die RGD-Stelle an Position 1097 kodiert.

8. Isoliertes Polypeptidfragment von FHA, für das der offene Leserahmen des Nukleinsäurefragments nach einem der Ansprüche 5 bis 7 kodiert.

9. Fragment einer Nukleinsäuresequenz nach einem der Ansprüche 1 bis 3, das für ein Peptid mit einer Länge von zumindest 12 Aminosäuren kodiert, wobei das Peptid im wesentlichen aus der Aminosäuresequenz E-A-R-K-D-E gemeinsam mit ihren benachbarten Sequenzen von bis zu 100 Aminosäuren in beide Richtungen besteht.

10. Isoliertes Polypeptid mit einer Länge von zumindest 12 Aminosäuren, das ein Fragment des B.pertussis-FHA-Proteins mit 3597 Aminosäuren ist und im wesentlichen aus der Aminosäuresequenz E-A-R-K-D-E gemeinsam mit ihren angrenzenden Sequenzen von bis zu 100 Aminosäuren in beiden Richtungen besteht.

11. Expressionskonstrukt, das die Nukleinsäure nach einem der Ansprüche 1 bis 3 oder das Fragment nach einem der Ansprüche 4 bis 7 oder 9 umfaßt, wobei die Nukleinsäure oder das Fragment operabel mit einem Transkriptionsinitiationsbereich und einem Transkriptionsterminationsbereich verbunden ist.

12. Prokaryotische Zelle, die transformiert ist mit:
(a) einer Nukleinsäure nach einem der Ansprüche 1 bis 3 oder einem Fragment davon nach einem der Ansprüche 4 bis 7 oder 9, wobei die Nukleinsäure oder das Fragment an eine andere DNA-Sequenz angefügt ist als die Nukleinsäuresequenz von B.pertussis;
(b) einem Expressionskonstrukt nach Anspruch 11.

13. Prokaryotische Zelle nach Anspruch 12, die B.pertussis ist.

14. Verfahren zur Herstellung eines Polypeptids, für das eine Nukleinsäure nach einem der Ansprüche 1 bis 3 oder ein Fragment davon nach einem der Ansprüche 4 bis 7 oder 9 kodiert, wobei das Verfahren umfaßt: das Kultivieren einer Wirtszelle nach Anspruch 12 oder 13 unter Bedingungen, die zur Expression des Polypeptids führen; sowie das Gewinnen des Polypeptids.

15. Impfstoff, der ein Polypeptid umfaßt, das von Antikörpern gegen das filamentöse Hemagglutinin von B.pertussis erkannt wird, das nach dem Verfahren von Anspruch 14 erhalten wird oder erhältich ist.

16. Verwendung eines Polypeptids, das nach dem Verfahren von Anspruch 14 erhalten wird oder erhältlich ist, bei der Herstellung eines Medikaments zur Verwendung bei einem Verfahren zur Behandlung oder Diagnose des Körpers eines Menschen oder Tiers.

## Revendications

1. Séquence d'acides nucléiques de moins de 15kb comprenant le gène *B.pertussis fhaB* de 10788 kb exempte du gène *FhaB*.

2. Séquence d'acides nucléiques de moins de 15kb comprenant une séquence de nucléotides encodant la protéine *B.pertussis* FHA de 3597 acides aminés.

3. Séquence d'acides nucléiques selon la revendication 1 ou 2 dans laquelle la séquence comprend les nucléotides 253 à 11040 comme décrit dans la description annexée .

4. Fragment d'une séquence d'acides nucléiques selon l'une quelconque des revendications précédentes encodant une portion d'approximativement 230KDa de la protéine FHA, ladite portion étant la portion proche du N de ladite protéine.

5. Fragment d'une séquence d'acides nucléiques selon l'une queiconque des revendications 1 à 3 qui encode un peptide d'au moins 12 acides aminés de longueur, ledit fragment étant un fragment du cadre de lecture ouvert du gène *FhaB* qui encode le peptide encodé par la séquence de la revendication 3, dans lequel lesdits nucléotides sont caractérisés par un motif sélectionné dans le groupe des:
a) nucléotides encodant les sites de reconnaissance de cellules RGD aux positions 1097 ou 2599 du polypeptide FHA ;
b) nucléotides encodant le site protéolytique RRARR à la position 1069 du polypeptide FHA ;
c) nucléotides encodant les répétats directs situés entre les nucléotides 1468 et 1746 du gène *FhaB* ;
d) nucléotides encodant le motif EARKDE des positions 1211 à 1216 du polypeptide FHA ;
e) nucléotides encodant le motif SKQDER aux positions 3075 à 3080 du polypeptide FHA ;
f) nucléotides encodant le répétat (PK)₅ lequel répétat commence au résidu 3477 du polypeptide FHA ; et
g) nucléotides encodant la séquence VEVVPRKVET démarrant aux positions 3319 et 3350 du polypeptide FHA .

6. Fragment selon la revendication 5 qui comprend au moins 100 paires de bases.

7. Fragment selon la revendication 5 ou 6 qui encode le site RGD à la position 1097 .

8. Fragment de polypeptide isolé de FHA encodé par le cadre de lecture ouvert du fragment d'acides nucléiques selon l'une quelconque des revendications 5 à 7.

9. Fragment d'une séquence d'acides nucléiques selon l'une quelconque des revendications 1 à 3 qui encode un peptide d'au moins 12 acides aminés de longueur, ledit peptide consistant essentiellement en la séquence d'acides aminés E-A-R-K-D-E avec ses séquences adjacentes jusqu'à 100 acides aminés dans l'une ou l'autre direction .

10. Polypeptide isolé d'au moins 12 acides aminés de longueur qui est un fragment de la protéine FHA *B.pertussis* de 3597 acides aminés et qui consiste essentiellement en la séquence d'acides aminés E-A-R-K-D-E avec ses séquences adjacentes jusqu'à 100 acides aminés dans l'une ou l'autre direction .

11. Construction d'expression qui comprend l'acide nucléique selon l'une quelconque des revendications 1 à 3 ou le fragment selon l'une quelconque des revendications 4 à 7 ou 9, ledit acide nucléique ou fragment étant ponté opérationnellement à une région d'initiation de transcription et une région de terminaison de transcription.

12. Cellule procaryotique transformée avec:
(a) un acide nucléique selon l'une quelconque des revendications 1 à 3 ou un fragment de celui-ci selon l'une quelconque des revendications 4 à 7 ou 9, ledit acide nucléique ou fragment étant joint à une séquence d'ADN différente d'une séquence d'acide nucléique de *B.pertussis* ; ou
(b) une construction d'expression selon la revendication 11.

13. Cellule prokaryotique selon la revendication 12 qui est *B.pertussis*.

14. Méthode de production d'un polypeptide encodé par un acide nucléique selon l'une quelconque des revendications 1 à 3 ou un fragment de celui-ci selon l'une quelconque des revendications 4 à 7 ou 9, ladite méthode comprenant la culture d'une cellule hôte selon la revendication 12 ou 13 dans des conditions qui résultent en l'expression du polypeptide; et récupération du polypeptide.

15. Vaccin comprenant un polypeptide reconnu par des anticorps à l'hémagglutinine filamenteuse de *B.pertussis* obtenu ou pouvant être obtenu par la méthode selon la revendication 14.

16. Utilisation d'un polypeptide obtenu ou pouvant être obtenu par la méthode de la revendication 14 dans la fabrication d'un médicament pour utilisation dans une méthode de traitement ou de diagnostic du corps humain ou animal.
